# EUROPEAN PATENT SPECIFICATION

(11) **EP 2 819 663 B1**
(45) Date of publication and mention of the grant of the patent: **05.07.2017**
(21) Application number: 13706549.6
(22) Date of filing: 28.02.2013
(51) Int. Cl.: A61K 31/185, A61K 31/195, A61P 25/16

(54) **NEW THERAPEUTIC APPROACHES FOR TREATING PARKINSON'S DISEASE**
NEUE THERAPEUTISCHE ANSÄTZE ZUR BEHANDLUNG VON MORBUS PARKINSON
NOUVELLES APPROCHES THÉRAPEUTIQUES POUR LE TRAITEMENT DE LA MALADIE DE PARKINSON

(30) Priority: 01.03.2012 WO PCT/EP2012/053565; 01.03.2012 WO PCT/EP2012/053568; 01.03.2012 WO PCT/EP2012/053570; 05.09.2012 EP 12306063; 05.09.2012 US 201261696992 P
(43) Date of publication of application: 07.01.2015
(73) Proprietor: Pharnext, 92130 Issy-les-Moulineaux (FR)
(72) Inventor: COHEN, Daniel, 92210 Saint Cloud (FR); NABIROCHKIN, Serguei, 92290 Châtenay Malabry (FR); CHUMAKOV, Ilya, 77000 Vaux le Pénil (FR); HAJJ, Rodolphe, 78100 St Germain en Laye (FR)
(74) Representative: Becker, Philippe
(86) International application number: PCT/EP2013/054026
(87) International publication number: WO 2013/127918

(56) References cited:
- EP-A1- 2 322 163
- EP-A2- 0 467 710
- WO-A1-2009/133128
- WO-A2-02/056892
- V. V. MYLLYLA ET AL: "Role of selegiline in combination therapy of Parkinson's disease", NEUROLOGY, vol. 47, no. Issue 6, Supplement 3, December 1996 (1996-12), pages 200S-209S, XP055062612, ISSN: 0028-3878, DOI: 10.1212/WNL.47.6_Suppl_3.200S
- JIMENEZ-SHAHED J ET AL: "Acamprosate Improves Impulse Control Disorders in Parkinson's Disease", NEUROLOGY, vol. 70, no. 11 Suppl 1, March 2008 (2008-03), page A288, XP055062517, 60TH ANNUAL MEETING OF THE AMERICAN-ACADEMY-OF-NEUROLOGY; CHICAGO, IL, USA
- ANITA MADAN ET AL: "Intrathecal Baclofen Therapy Slows Progressive Disability in Multiple System Atrophy", NEUROMODULATION: TECHNOLOGY AT THE NEURAL INTERFACE, vol. 14, no. 2, March 2011 (2011-03), pages 176-178, XP055062528, ISSN: 1094-7159, DOI: 10.1111/j.1525-1403.2011.00336.x
- LINAZASORO G ET AL: "Pharmacological treatment of Parkinsonâ s disease: life beyond dopamine D2/D3 receptors?", JOURNAL OF NEURAL TRANSMISSION ; BASIC NEUROSCIENCES, GENETICS AND IMMUNOLOGY, PARKINSON'S DISEASE AND ALLIED CONDITIONS, ALZHEIMER'S DISEASE AND ADOLESCENT PSYCHIATRY RELATED DISORDERS, BIOLOGICAL PSYCHIATRY, BIOLOGICAL CHILD AND ADOLESCENT PSYCHIAT, vol. 115, no. 3, 11 February 2008 (2008-02-11), pages 431-441, XP019592403, ISSN: 1435-1463
- FOX S H ET AL: "Non-dopaminergic treatments in development for Parkinson's disease", LANCET NEUROLOGY, LANCET PUBLISHING GROUP, LONDON, GB, vol. 7, no. 10, October 2008 (2008-10), pages 927-938, XP025428983, ISSN: 1474-4422, DOI: 10.1016/S1474-4422(08)70214-X [retrieved on 2008-09-15]

## Description

### FIELD OF THE INVENTION

The present invention relates to compositions for the treatment of Parkinson's disease and related disorders. More specifically, the present invention relates to novel combinatorial therapies of Parkinson's disease and related disorders.

### BACKGROUND OF THE INVENTION

Parkinsonism or Parkinsonian syndromes are a group of progressive, multicentric neurodegenerative disorders whose main features are tremor at rest, rigidity, bradykinesia and postural instability. Parkinson's disease (PD) is the most common form of Parkinsonism and the second most common neurodegenerative disorder after Alzheimer's disease. In industrial countries, the prevalence of PD has been estimated at approximately 0.3% of the general population, the elderly being the most at risk (4% of the population over 80 are estimated to be affected). The mean age of onset is around 60 years, although early onset (as young as 20 year old) can occur (1).

PD is often classified as a movement disorder. Rest tremor is the most common and usually among the earliest symptoms to develop. Bradykinesia also usually appears in the early stages with difficulties performing tasks such as writing or getting dressed. Hyperkinetic movement disorders have been reported as side-effects of some treatments of Parkinson's disease. In this regard, US5,952,389 patent discloses the use of acamprosate for alleviating levodopa-induced hyperkinetic movement disorders. Limiting secondary effects of drugs is, however, distinct and remote from treating the disease or related symptoms. Rigidity occurs and progresses to stiffness and resistance to movement of the whole body, reducing the ability to move. In the late stages, the disease progresses to postural instability, leading to impaired balance and frequent falls. Other motor symptoms such as gait or swallowing disturbances can arise. If not treated, motor symptoms can lead to the patient being bedridden after an average of ten years (2, 3).

In later stages of the disease, PD gives rise to many non-motor symptoms which vary greatly individually. Disability is then greatly worsened by the development of autonomic and neuropsychiatric disturbances. Disorders of speech, cognition, mood, behavior, and/or thought will develop, leading eventually to dementia. Other common symptoms include sensory, sleep and emotional problems. Those disorders decrease the life expectancy of the individual affected and the mortality ratios are around twice those of people without PD (2-4).

PD is an idiopathic disease and its pathophysiology also remains poorly understood (4). However, at least 5% of PD cases can be attributed to genetic variations. Mutations within genes such as SNCA (alpha-synuclein), PRKN (parkin), LRRK2 (leucine-rich repeat kinase 2), PINK1 (PTEN-induced putative kinase 1), DJ-1 and ATP13A2 and eleven gene loci (PARK1-PARK11) have been associated with familial PD (5). DJ1 is suspected to be an ubiquitous redox-responsive cytoprotective protein thereby confirming the pivotal role of oxidative stress in PD (28), that is further evidenced by the protective role of Hypoxia Inducible Factor in nigral dopaminergic cell protection against oxidative stress, mitochondrial dysfunction and iron homeostasis disturbance (29). Apart from genetic factors, many environmental risk factors have been proposed to be involved in the onset of PD but none with undisputed evidence. The most frequently replicated risk factor is exposure to metals, pesticides or herbicides such as Agent Orange. On another hand, smoking and caffeine consumption seem to protect individuals from PD (1).

The pathophysiology of PD is characterized by four features (4):
(i) A synucleinopathy characterized by the abnormal accumulation of alpha-synuclein protein into inclusions called Lewy bodies in the brain. The distribution of the Lewy bodies throughout the brain varies from one individual to another but is often directly associated with the expression and degree of the clinical symptoms.
(ii) Glutamate is the most abundant excitatory neurotransmitter in the mammalian nervous system. Under pathological conditions, its abnormal accumulation in the synaptic cleft leads to glutamate receptors overactivation. Abnormal accumulation of glutamate in synaptic cleft leads to the overactivation of glutamate receptors that results in pathological processes and finally in neuronal cell death. This process, named excitotoxicity, is commonly observed in neuronal tissues during acute and chronic neurological disorders. It is becoming evident that excitotoxicity is involved in the pathogenesis of Parkinson's disease.
(iii) A dopaminergic activity deficiency due to the death of dopamine-generating cells in the substantia nigra, a region of the midbrain. This results in a loss of muscle movement and tone control, leading to the motor symptoms of PD.
(iv) Degeneration of NANC (non-adrenergic, non-cholinergic), serotonergic and cholinergic neurons also occurs in later stages of the disease, leading to the non-motor symptoms of PD.

As no biological tests are available, diagnosis of PD is mainly based on observation of clinical symptoms and exclusion of other disorders with similar clinical features (3). Postmortem confirmation is required for a definitive diagnosis. Neurological examination by neuroimaging can be useful to detect changes in dopaminergic neurons and to rule out other diseases. Positive therapeutic response to levodopa is another diagnosis criterion. Once the diagnosis made, the progression and severity of the disease is rated using a stages scale such as the Unified Parkinson's Disease Rating Scale.

The most widely used treatment, especially at earlier stages, is the dopamine precursor levodopa (L-DOPA) (6). The drug brings the lacking neurotransmitter to the dopaminergic neurons, thus decreasing motor symptoms. However, most of the drug is metabolized before to reach the blood brain barrier, causing a variety of side effects, especially gastrointestinal effects (such as anorexia, nausea or vomiting), dyskinesia and psychiatric symptoms (7). To prevent dyskinesia phenomenon, L-DOPA is therefore usually given in combination with carbidopa or benserazide (peripheral dopa decarboxylase inhibitors) and often also with catechol-O-methyl transferase inhibitors such as entacapone. These drugs aim at preventing L-DOPA metabolism before to reach the brain, enhancing the activity of the drug (6). Although less effective at improving motor symptoms, dopamine agonists such as pergolide, cabergoline, apomorphine or lisuride and monoamine oxidase-B inhibitors (involved in the catabolic breakdown of dopamine) such as selegiline or rasagiline are commonly used at early stages of the disease. Although less effective, they may be useful at delaying the use of levodopa and thus the onset of dyskinesia (7).

Other drugs such as anticholinergics and nicotinic acetylcholine receptor agonists may be useful but their efficacy for PD remains to be confirmed (7). Current research also focuses on neuroprotective treatments, but none of them provided evidence of improved degeneration. They target apoptosis (omigapil, CEP-1347), glutamate receptors, adenosine A2A receptor, calcium channels (isradipine), growth factors (GDNF), alpha-synuclein and inflammation (8).

Ongoing pharmaceutical research has shown a growing interest on gene therapy and neural transplantation (8).

WO 2009/133128, WO 2009/133141, WO 2009/133142, WO 2011/054759, EP2322163, WO 2009/068668, WO 2009/153291 disclose potential treatments for several neurodegenerative diseases, among which, PD. Linazasoro et al. discusses non-dopaminergic treatments of PD (34). Jimenez-Shahed et al (35) and Fox et al (36) mention that acamprosate may potentially improve Impulse Control Disorders behaviour. Madan and Schiess (37) suggest that intrathecal baclofen therapy may slow progressive disability in multiple system atrophy.

PD remains so far an incurable disease and no effective disease-modifying treatment has been discovered yet. Therefore, current treatments aim at relieving symptoms and alleviate the slow progression of the disease.

### SUMMARY OF INVENTION

The present invention relates to new therapeutic compositions for treating Parkinsonism. The invention stems, inter alia, from the identification of drug combinations which provide improved therapeutic effect and clinical benefit to subjects having Parkinsonism condition, particularly subjects having Parkinson's disease.

More particularly, the invention discloses a composition for use in the treatment of Parkinsonism, particularly Parkinson's disease, comprising one, preferably at least two drugs selected from acamprosate, baclofen, cinacalcet, mexiletine, sulfisoxazole and torasemide, or a salt, prodrug, derivative of any chemical purity, or sustained-release formulation thereof.

An object of this invention relates to a composition as defined in the claims.

This invention discloses a method for treating Parkinsonism, particularly Parkinson's disease, in a subject in need thereof, comprising administering to the subject at least two drugs selected from acamprosate, baclofen, cinacalcet, mexiletine, sulfisoxazole and torasemide, or a salt, prodrug, derivative of any chemical purity, or sustained-release formulation thereof.

Preferred drug combinations for use in the invention comprise baclofen and acamprosate. In a particular embodiment, the compositions further comprise Levodopa.

The compositions in the invention may further comprise one or several pharmaceutically acceptable carrier(s) or excipient(s), and they may be administered repeatedly to the subject. Preferred compositions are administered orally. Moreover, the drugs may be formulated or administered together, separately or sequentially.

The invention is suitable for treating Parkinsonism in any mammalian subject, particularly in a human subject, at any stage of the disease. The invention may be used *e.g*., to retard the development of the disease, to reduce, delay or prevent tremor, hypokinesia (*e.g*., bradykinesia, akinesia, rigidity) postural instability, and/or pain, and/or to increase survival.

### BRIEF DESCRIPTION OF THE FIGURES

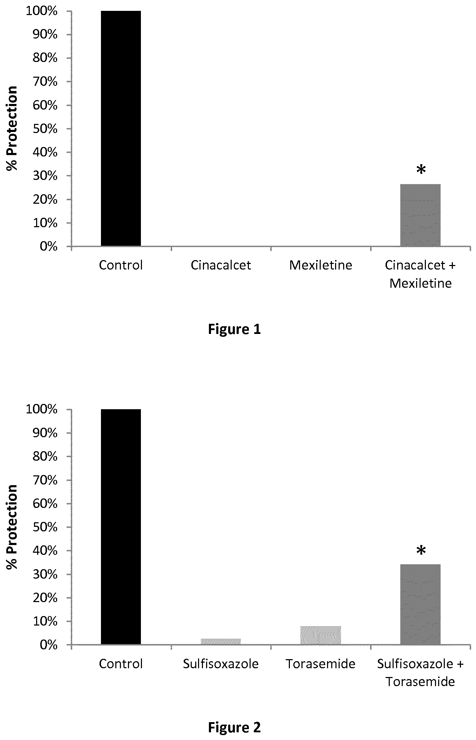

**Figure 1****:** Effect of cinacalcet and mexiletine combination therapy against glutamate toxicity on neuronal cortical cells. The glutamate intoxication is significantly prevented by the combination of cinacalcet (64 pM) and mexiletine (25.6 pM) whereas, at those concentrations, cinacalcet and mexiletine alone have no significant effect on intoxication. *: p<0.001, significantly different from glutamate intoxication; (ANOVA + Dunnett Post-Hoc test).
**Figure 2****:** Effect of sulfisoxazole and torasemide combination therapy against glutamate toxicity on neuronal cortical cells. The glutamate intoxication is significantly prevented by the combination of sulfisoxazole (6.8 nM) and torasemide (400 nM) whereas, at those concentrations, sulfisoxazole and torasemide alone have no significant effect on intoxication.*: p<0.001, significantly different from glutamate intoxication; (ANOVA + Dunnett Post-Hoc test).

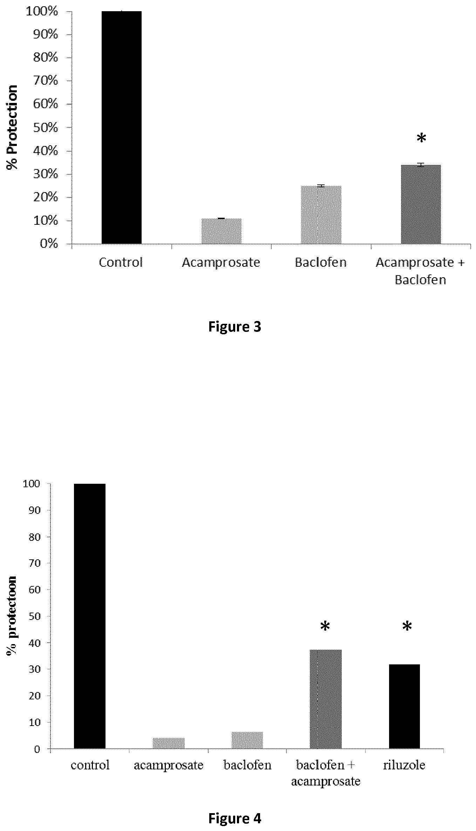

**Figure 3****:** Effect of baclofen and acamprosate combination therapy against glutamate toxicity on neuronal cortical cells glutamate intoxication is significantly prevented by the combination of baclofen (400 nM) and acamprosate (1.6 nM) whereas, at those concentrations, baclofen and acamprosate alone have no significant effect on intoxication. *: p<0.001, significantly different from glutamate intoxication; (ANOVA+ Dunnett Post-Hoc test).
**Figure 4****:** Protective effect of baclofen and acamprosate combination against ischemic injury. Whereas no significant protection is obtained when baclofen (80 nM) or acamprosate (0.32 nM) are used alone, a significant protection (*: p<0.0001) is observed for the combination of the two drugs, at the same concentrations.

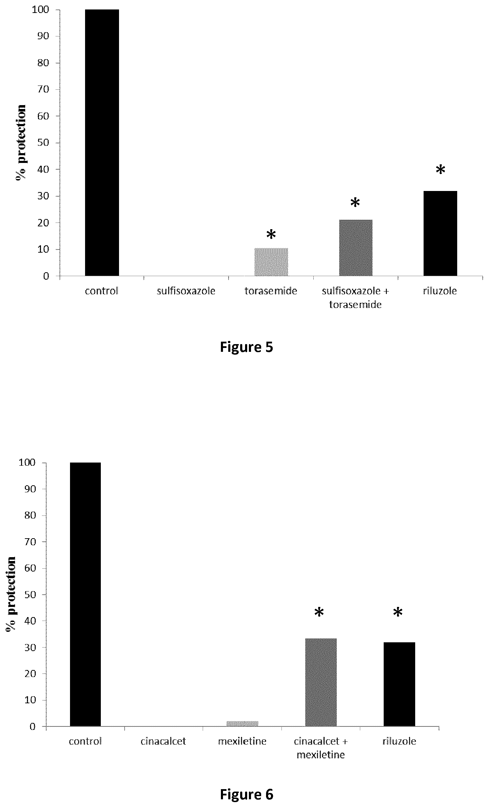

**Figure 5****:** Protective effect of cinacalcet and mexiletine combination against ischemic injury. No significant protection is observed when cinacalcet (64 pM) or mexiletine (25.6 pM) are used alone, whereas a significant protection (*: p<0.0001) is observed for the combination of the two drugs, at the same concentrations.
**Figure 6****:** Protective effect of torasemide and sulfisoxazole combination against ischemic injury. The combination of sulfisoxazole (1.36 nM) and torasemide (80 nM) induce a significant protection (*: p<0.0001), 110% higher than the protection obtained using torasemide alone, whereas no protection is obtained when sulfisoxazole is used alone.

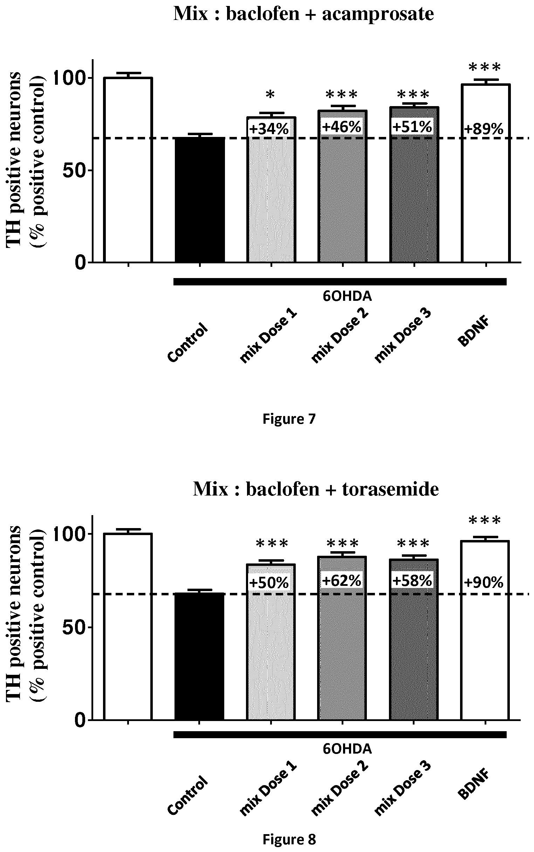

**Figure 7****:** Effect of baclofen and acamprosate combination therapy against 6OHDA injury on dopaminergic neuronal cells. The protection increases correlatively with concentration of mixes. A significant protective effect is observed with an increase in TH neurons survival by 34% with dose 1 (16 nM and 64 pM respectively), by 46% with dose 2 (80 nM and 144 pM) and by 51 % with dose 3 (400 nM and 1600 pM) (***: p<0.0001; *: p<0.001: significantly different from 6OHDA intoxicated cells (ANOVA + Dunnett test)).
**Figure 8****:** Effect of baclofen and torasemide combination therapy against 6OHDA injury on dopaminergic neuronal cells. A significant protective effect is observed with an increase in TH neurons survival by 50% with low dose 1 (80 nM and 16 nM respectively), by 62% with the middle dose 2 (240 nM and 48 nM) and by 58% with high dose 3 (720 nM and 144 nM) (***: p<0.0001: significantly different from 6OHDA intoxicated cells (ANOVA + Dunnett test)).

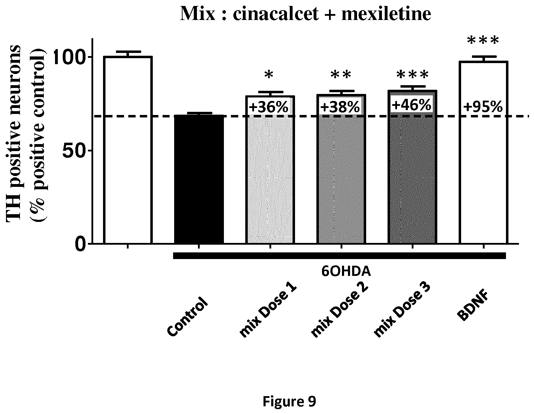

**Figure 9****:** Effect of cinacalcet and mexiletine combination therapy against 6OHDA injury on dopaminergic neuronal cells. All the tested concentrations afford a significant protection againt 6OHDA. Indeed, a significant protective effect is observed with an increase in TH neurons survival by 36% with dose 1 (64 pM and 5 pM respectively), by 38% with the dose 2 (64 pM and 26 pM) and by 48% with dose 3 (1600 pM and 64 pM) (***: p<0.0001; *: p<0.001: significantly different from 6OHDA intoxicated cells (ANOVA + Dunnett test)).

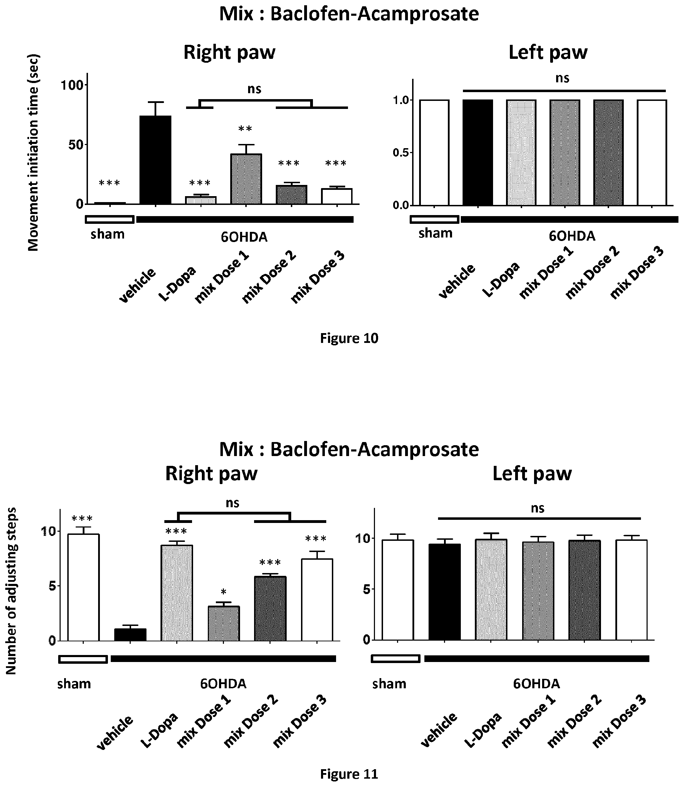

**Figure 10****:** Initiation time test, effect of baclofen and acamprosate combination therapy against 6OHDA stereotaxic lesion in the left *substantia nigra pars compacta.* Left paw: no significant change. Right paw: 6OHDA injection strongly prolonged initiation time as a result of death of neurons in the left *substantia nigra.* Baclofen-acamprosate treatment strongly protects from 6OHDA induced akinesia and this from the weakest dose 1 (baclofen-acamprosate dose 1: 0.6 mg/kg/bid and 0.04 mg/kg/bid respectively ; dose 2 : 1.5 mg/kg/bid and 0.1 mg/kg/bid ; dose 3: 3.75 mg/kg/bid and 0.25 mg/kg/bid ; ***: p<0.0001; **: p<0.001; *: p<0.05: significantly different from 6OHDA intoxicated cells (ANOVA + Dunnett test)).
**Figure 11****:** Reaction time test, effect of baclofen and acamprosate combination therapy against 6OHDA stereotaxic lesion in the left *substantia nigra pars compacta.* Left paw: no significant change. Right paw: 6OHDA injection strongly prolonged reaction time as a result of death of neurons in the left *substantia nigra.* Baclofen-acamprosate treatment strongly protects from 6OHDA induced akinesia and this from the weakest dose 1. Dose 2 and 3 almost fully alleviate 6OHDA induced akinesia (baclofen-acamprosate dose 1: 0.6 mg/kg/bid and 0.04 mg/kg/bid ; dose 2: 1.5 mg/kg/bid and 0.1 mg/kg/bid ; dose 3: 3.75 mg/kg/bid and 0.25 mg/kg/bid; ***: p<0.0001; **: p<0.001; *: p<0.05: significantly different from 6OHDA intoxicated cells (ANOVA + Dunnett test)).

### DETAILED DESCRIPTION OF THE INVENTION

It is an object of the present invention to provide new therapeutic approaches for treating Parkinsonism, more specifically Parkinson's disease. More particularly the invention discloses novel use of drugs and drug combinations and methods, which allow an effective correction of such diseases and may be used in any mammalian subject.

Parkinsonism defines a group of progressive neurodegenerative disorders characterized by tremor at rest and/or bradykinesia, associated with rigidity, postural instability, loss of postural reflexes, flexed posture, and/or the freezing phenomenom (when the feet are transiently "glued" to the ground). Examples of Parkinsonism conditions include Parkinson's disease, progressive supranuclear palsy, multiple system atrophy, cortical-basal ganglionic degeneration, diffuse Lewy body disease, Parkinson-dementia, X-linked dystonia-parkinsonism, and secondary Parkinsonism (resulting from environmental etiology, *e.g*., toxins, drugs, encephalitis, brain tumors, head trauma, normal pressure hydrocephalus).

Parkinson's disease is the most common form of Parkinsonism. Parkinson's Disease ("PD") is a neurodegenerative disorder leading to motor and non-motor manifestations and characterized by extensive degeneration of dopaminergic neurons in the nigrostriatal system. The motor manifestations of PD are attributable to the degeneration of dopaminergic neurons within the *substantia nigra.* They include tremor, hypokinesia (*e.g*., bradykinesia, akinesia, rigidity) postural instability, abnormal gait and swallowing disturbances. Non-motor symptoms include autonomic and neuropsychiatric disturbances such as anosmia, or sleep abnormalities. Within the context of the invention, the term PD includes any of the above manifestations of the disease.

As used herein, "treatment" includes the therapy, prevention, prophylaxis, retardation or reduction of symptoms provoked by or of the causes of Parkinsonism, preferably of Parkinson's disease. The term treatment also designates a retardation or delayed onset of tremor, a reduction of pain, a decrease or reduction of bradykinesia, akinesia, rigidity, postural instability, abnormal gait, anosmia, and/or sleep abnormalities, and/or an increase of survival. The term treatment includes in particular the control of disease progression and associated motor and non-motor symptoms. The term treatment particularly includes i) a protection against the toxicity caused by alpha-synuclein, or a reduction or retardation of said toxicity, and/or ii) a protection of dopaminergic neurons against the toxicity resulting from abnormal glutamate accumulation, oxidative stress, mitochondrial dysfunction or neuroinflammation, or a reduction or retardation of said toxicity, in the treated subjects.

The term "combination or combinatorial treating/therapy" designates a treatment wherein at least two or more drugs are co-administered to a subject to cause a biological effect. In a combined therapy according to this invention, the at least two drugs may be administered together or separately, at the same time or sequentially. Also, the at least two drugs may be administered through different routes and protocols. As a result, although they may be formulated together, the drugs of a combination may also be formulated separately.

Several biological processes such as oxidative stress, mitochondrial dysfunction and neuroinflammation accompany accumulation of aggregated alpha-synuclein which leads to the degeneration of dopaminergic neurons. On the other hand, abnormal accumulation of glutamate in synaptic cleft leads to the overactivation of glutamate receptors that results in pathological processes and finally in neuronal cell death. This process known as excitotoxicity is now recognized as an important etiological factor implicated in development of Parkinson's disease.

The inventors were able to establish a network underlying alpha-synuclein aggregation which is a major functional network affected in Parkinson's disease. The inventors have identified functional modules composed of several target proteins, within the alpha-synuclein aggregation network. Such proteins are functionally relevant to the genesis and control of Parkinson's disease and Parkinsonism, and represent valuable targets for therapies and particularly combination therapies.

Hence, the invention discloses to the use of particular drugs which, alone or preferentially in combination(s), modulate the above pathways to treat Parkinsonism, particularly Parkinson's disease.

In a particular embodiment, the present invention more specifically relates to compositions using a drug combination that inhibits the activity of at least two distinct proteins involved in alpha-synuclein aggregation network. The therapeutic approaches of the invention are effective for the protection of neuronal cells, particularly for the protection of dopaminergic neurons in the midbrain and more particularly in the *substantia nigra.*

More particularly, the invention discloses a composition for use in the treatment of Parkinsonism, particularly Parkinson's disease (PD), comprising at least two drugs selected from acamprosate, baclofen, cinacalcet, mexiletine, sulfisoxazole and torasemide, or salts or prodrugs or derivatives of any purity or sustained release formulations thereof.

Indeed, the inventors have surprisingly found that these compounds show a protective activity against glutamate toxicity, which is a known cause of neuronal death in Parkinson's disease and Parkinsonism. The compounds and combination therapies of the invention also show a protective activity against ischemic stress which share common physiological features with Parkinson's disease (notably mitochondrial dysfunction and oxidative stress). More particularly compounds of the present invention are particularly efficient *in vivo* and *in vitro* against oxidative stress which is one component of alpha-synuclein toxicity for dopaminergic neurons.

The invention also discloses a method for the treatment of Parkinsonism, particularly Parkinson's disease (PD), comprising administering to a subject in need thereof at least two compounds selected from acamprosate, baclofen, cinacalcet, mexiletine, sulfisoxazole and torasemide, or salts or prodrugs or derivatives of any purity or sustained release formulations thereof.

The term "prodrug" as used herein refers to any functional derivatives (or precursors) of a compound of the present invention, which, when administered to a biological system, generates said compound as a result of *e.g*., spontaneous chemical reaction(s), enzyme catalysed chemical reaction(s), and/or metabolic chemical reaction(s). Prodrugs are usually inactive or less active than the resulting drug and can be used, for example, to improve the physicochemical properties of the drug, to target the drug to a specific tissue, to improve the pharmacokinetic and pharmacodynamic properties of the drug and/or to reduce undesirable side effects. Some of the common functional groups that are amenable to prodrug design include, but are not limited to, carboxylic, hydroxyl, amine, phosphate/phosphonate and carbonyl groups. Prodrugs typically produced via the modification of these groups include, but are not limited to, esters, carbonates, carbamates, amides and phosphates. Specific technical guidance for the selection of suitable prodrugs is general common knowledge (29-33). Furthermore, the preparation of prodrugs may be performed by conventional methods known by those skilled in the art. Methods which can be used to synthesize other prodrugs are described in numerous reviews on the subject (9; 14-20). For example, arbaclofen placarbil is listed in ChemID plus Advance database (website: chem.sis.nlm.nih.gov/chemidplus/) and arbaclofen placarbil is a well-known prodrug of baclofen (21-22). Specific examples of prodrugs of baclofen are given in Hanafi *et al.,* 2011 (26), particularly baclofen esters and baclofen ester carbamates, which are of particular interest for CNS targeting. Hence such prodrugs are particularly suitable for compositions of this invention. Arbaclofen placarbil as mentioned before is also a well-known prodrug and may thus be used instead of baclofen in compositions of the invention. Other prodrugs of baclofen can be found in the following patent applications: WO2010102071, US2009197958, WO2009096985, WO2009061934, WO2008086492, US2009216037, WO2005066122, US2011021571, WO2003077902, and WO2010120370.

Useful prodrugs for acamprosate such as pantoic acid ester neopentyl sulfonyl esters, neopentyl sulfonyl esters prodrugs or masked carboxylate neopentyl sulfonyl ester prodrugs of acamprosate are notably listed in WO2009033069, WO2009033061, WO2009033054 WO2009052191, WO2009033079, US 2009/0099253, US 2009/0069419, US 2009/0082464, US 2009/0082440, and US 2009/0076147.

Prodrugs as described above can be used instead of the herein disclosed compounds of the invention.

The term "derivative" of a compound includes any molecule that is functionally and structurally related to said compound, such as an acid, amide, ester, ether, acetylated variant, hydroxylated variant, or an alkylated (C1-C6) variant of such a compound. The term derivative also includes structurally related compound having lost one or more substituent as listed above. For example, homotaurine is a deacetylated derivative of acamprosate. Preferred derivatives of a compound are molecules having a substantial degree of similarity to said compound, as determined by known methods. Similar compounds along with their index of similarity to a parent molecule can be found in numerous databases such as PubChem (http://pubchem.ncbi.nlm.nih.gov/search/) or DrugBank (http://www.drugbank.ca/). In a more preferred embodiment, derivatives should have a Tanimoto similarity index greater than 0.4, preferably greater than 0.5, more preferably greater than 0.6, even more preferably greater than 0.7 with a parent drug. The Tanimoto similarity index is widely used to measure the degree of structural similarity between two molecules. Tanimoto similarity index can be computed by software such as the Small Molecule Subgraph Detector (23-24) available online (http://www.ebi.ac.uk/thornton-srv/software/SMSD/). Preferred derivatives should be both structurally and functionally related to a parent compound, *i.e.,* they should also retain at least part of the activity of the parent drug, more preferably they should have a protective activity on dopaminergic neurons from 6OHDA-induced stress and/or glutamate toxicity and/or ischemic stress (as exemplified in the experimental part).

The term derivatives also includes metabolites of a drug, *e.g*., a molecule which results from the (biochemical) modification(s) or processing of said drug after administration to an organism, usually through specialized enzymatic systems, and which displays or retains a biological activity of the drug. Metabolites have been disclosed as being responsible for much of the therapeutic action of the parent drug. In a specific embodiment, a "metabolite" as used herein designates a modified or processed drug that retains at least part of the activity of the parent drug, preferably that has a protective activity on dopaminergic neurons from 6OHDA-induced stress and/or glutamate toxicity and/or ischemic stress. Examples of metabolites include hydroxylated forms of torasemide resulting from the hepatic metabolism of the drug (27).

The term "salt" refers to a pharmaceutically acceptable and relatively non-toxic, inorganic or organic acid addition salt of a compound of the present invention. Pharmaceutical salt formation consists in pairing an acidic, basic or zwitterionic drug molecule with a counterion to create a salt version of the drug. A wide variety of chemical species can be used in neutralization reaction. Pharmaceutically acceptable salts of the invention thus include those obtained by reacting the main compound, functioning as a base, with an inorganic or organic acid to form a salt, for example, salts of acetic acid, nitric acid, tartric acid, hydrochloric acid, sulfuric acid, phosphoric acid, methane sulfonic acid, camphor sulfonic acid, oxalic acid, maleic acid, succinic acid or citric acid. Pharmaceutically acceptable salts of the invention also include those in which the main compound functions as an acid and is reacted with an appropriate base to form, *e.g*., sodium, potassium, calcium, magnesium, ammonium, or choline salts. Though most of salts of a given active principle are bioequivalents, some may have, among others, increased solubility or bioavailability properties. Salt selection is now a common standard operation in the process of drug development as taught by H. Stahl and C.G Wermuth in their handbook (25).

In a preferred embodiment, the designation of a compound is meant to designate the compound *per se,* as well as any pharmaceutically acceptable salt, hydrate, isomer, racemate thereof.

Table I below provides non limiting examples of CAS number of compounds for use in the invention as well as of salt(s), derivatives, metabolites, and/or prodrugs of these compounds.

**Table 1**

| **Drug** | **CAS Numbers** | **Class or Tanimoto similarity index** |
|---|---|---|
| acamprosate and related compounds | | |
| acamprosate | 77337-76-9; 77337-73-6 | NA |
| homotaurine | 3687-18-1 | 0.73 |
| Ehyl Dimethyl Ammonio Propane Sulfonate | / | 0.77 |
| taurine | 107-35-7 | 0.5 |

| baclofen and related compounds | | |
|---|---|---|
| baclofen | 1134-47-0; 66514-99-6; 69308-37-8; 70206-22-3; 63701-56-4; 63701-55-3; 28311-31-1 | NA |
| 3-(p-chlorophenyl)-4-hydroxybutyric acid | / | Metabolite |
| arbaclofen placarbil | 847353-30-4 | Prodrug |

| mexiletine and related compounds | | |
|---|---|---|
| mexiletine | 31828-71-4; 5370-01-4 | |
| 6-hydroxymethylmexiletine | 53566-98-6 | Metabolite |
| 4-hydroxymexiletine | 53566-99-7 | Metabolite |
| 3-hydroxymexiletine (MHM) | 129417-37-4 | Metabolite |
| N-hydroxymexiletine glucuronide | 151636-18-9 | Metabolite |

| sulfisoxazole and related compounds | | |
|---|---|---|
| sulfisoxazole | 127-69-5 ; 4299-60-9 | |
| N(4)-acetylsulfisoxazole | 4206-74-0 | Metabolite |
| sulfisoxazole acetyl | 80-74-0 | Prodrug |
| sulfamethoxazole | 723-46-6 | 0.52 |

| cinacalcet and related compounds | | |
|---|---|---|
| cinacalcet | 226256-56-0; 364782-34-3 | |
| hydrocinnamic acid | 501-52-0 | Metabolite |

| torasemide and related compounds | | |
|---|---|---|
| torasemide | 56211-40-6 ;72810-59-4 | |
| hydroxytorasemide | 99300-68-2; 99300-67-1 | Metabolites |
| carboxytorasemide | | Metabolite |
| tolbutamide | 64-77-7 | 0.55 |

In a particular embodiment, a sustained-release formulation of the compound is used.

The inventors have discovered that acamprosate, baclofen, cinacalcet, mexiletine, sulfisoxazole and torasemide are particularly efficient in correcting alpha-synuclein aggregation molecular pathways.

As disclosed in the examples, molecules of the invention have a strong, unexpected effect on biological processes involved in Parkinsonism, particularly Parkinson's disease, and represent new therapeutic approaches of the pathology. In particular, compositions of the invention provide an unexpected effect on glutamate toxicity. Furthermore, drugs and drug combinations of the invention increase the dopaminergic neuron survival under 6OHDA-induced oxidative stress as well as ischemic stress, and induce a protective effect on motor and non-motor manifestations of PD. Thus, therapeutic approaches of the invention are effective for the protection of neuronal cells, particularly for the protection of dopaminergic neurons in the midbrain and more particularly in the *substantia nigra* as shown *in vivo.*

In a particular embodiment, the present invention discloses compositions for treating Parkinsonism, particularly Parkinson's disease using a compound selected from acamprosate, baclofen, cinacalcet, mexiletine, sulfisoxazole and torasemide.

Drug combinations that modulate the activity of at least two distinct proteins which are involved in the alpha-synuclein aggregation network constitute a particularly advantageous embodiment of the invention. Indeed, the inventors have observed that the above drugs, when administered in combination, act synergistically to efficiently protect dopaminergic neurons. In particular, compositions of the invention have an unexpected effect on glutamate toxicity, ischemia induced cell death and oxidative stress. Such a strong and unexpected effect on biological processes involved in Parkinsonism, particularly Parkinson's disease make these new combinatorial therapeutic approaches of the pathology of particular interest.

The invention thus discloses such compositions for treating Parkinsonism, particularly Parkinson's disease using at least two drugs selected from acamprosate, baclofen, cinacalcet, mexiletine, sulfisoxazole and torasemide.

The use of compositions of the invention lead to an improvement of PD through their action on motor as well as non-motor symptoms of the disease. Therapeutic approaches of the invention provide an efficient neuronal protection, particularly of dopaminergic neurons, against oxidative stress, mitochondrial dysfunction, excitotoxicity damages, neuroinflammation or apoptosis. More particularly, they can provide a protection of the *substantia nigra* neurons against the toxicity of aggregated alpha-synuclein to reduce the rate or extent of dopaminergic cell loss and thereby affect the course of the disease progression.

In this regard, the invention discloses a composition for use in the treatment of Parkinsonism, particularly PD, comprising a compound selected from the group consisting of acamprosate, baclofen, cinacalcet, mexiletine, sulfisoxazole and torasemide, or salts or prodrugs or derivatives of any purity or sustained release formulations thereof. The invention also discloses the composition comprising torasemide or mexiletine, or a salt or prodrug or derivative of any purity or sustained release formulation thereof.

The invention also discloses a composition for use in the treatment of Parkinsonism, particularly PD, comprising at least two compounds selected from acamprosate, baclofen, cinacalcet, mexiletine, sulfisoxazole and torasemide, or a salt, prodrug, derivative of any chemical purity, or sustained release formulations thereof.

The invention also discloses a composition for use in the treatment of Parkinsonism, particularly PD, comprising at least one compound selected from acamprosate, cinacalcet, and torasemide, or a salt, prodrug, derivative of any chemical purity, or sustained release formulations thereof, and at least one compound selected from baclofen, mexiletine, or sulfisoxazole, or a salt, prodrug, derivative of any chemical purity, or sustained release formulations thereof.

More particularly, the invention discloses a composition comprising at least one of the following drug combinations, for simultaneous, sequential or separate administration:
- baclofen and acamprosate,
- baclofen and cinacalcet,
- cinacalcet and acamprosate,
- mexiletine and cinacalcet,
- torasemide and baclofen, or
- torasemide and sulfisoxazole,
or salt(s) or prodrug(s) or derivative(s) of any purity or sustained release formulations thereof for use in the treatment of Parkinsonism, particularly Parkinson's disease.

A particularly preferred composition of the invention comprises baclofen and acamprosate, or a salt, or sustained release formulations thereof.

Another composition disclosed in this invention uses baclofen and torasemide, or a salt, prodrug, derivative of any chemical purity, or sustained release formulations thereof.

Another composition disclosed in this invention uses acamprosate and cinacalcet, or salt(s) or prodrug(s) or derivative(s) of any purity or sustained release formulations, wherein the daily dosage of acamprosate is equal or lower to 10 mg.

The invention also relates to baclofen, or a salt, or sustained release formulations thereof, for use in combination with acamprosate, or a salt, or sustained release formulations thereof, for the treatment of Parkinsonism, particularly Parkinson's disease by combined, separate or sequential administration to a subject.

Preferred drug compositions of the invention therefore comprise 2, 3, 4 or 5 distinct drugs, more preferably 2, 3 or 4 distinct drugs for combinatorial treatment of Parkinsonism, particularly Parkinson's disease in a subject in need thereof. In a preferred embodiment, the drugs of the invention are used in combination(s) for combined, separate or sequential administration, in order to provide the most effective effect.

The inventors further discovered that combination of at least one drug combination selected from the group consisting of:
- baclofen and acamprosate,
- baclofen and cinacalcet,
- mexiletine and cinacalcet,
- cinacalcet and acamprosate,
- torasemide and baclofen, or
- torasemide and sulfisoxazole,
with a drug, different from previous, selected from drug acamprosate, baclofen, cinacalcet, mexiletine, sulfisoxazole and torasemide, and which enhances the therapeutic effect of the binary combination and leads to even more efficient compositions for use in the treatment of Parkinsonism, particularly PD.

Thus, the invention also discloses a composition for use in the treatment of Parkinsonism, particularly PD, comprising cinacalcet and acamprosate in a combination with a drug selected from drug baclofen, mexiletine, sulfisoxazole and torasemide or salts, prodrugs, derivatives or sustained release formulations thereof, for combined, separate or sequential administration.

The invention also relates to a composition for use in the treatment of Parkinsonism, particularly PD, comprising baclofen and acamprosate in a combination with a drug selected from cinacalcet, mexiletine, sulfisoxazole and torasemide or salts, or derivatives or sustained release formulations thereof, for combined, separate or sequential administration.

The invention further discloses a composition for use in the treatment of Parkinsonism, particularly PD, comprising baclofen and cinacalcet in a combination with a drug selected from acamprosate, mexiletine, sulfisoxazole and torasemide or salts, prodrugs, derivatives or sustained release formulations thereof, for combined, separate or sequential administration.

The invention also discloses a composition for use in the treatment of Parkinsonism, particularly PD, comprising mexiletine and cinacalcet in a combination with a drug selected from acamprosate, baclofen, sulfisoxazole and torasemide or salts, prodrugs, derivatives or sustained release formulations thereof, for combined, separate or sequential administration.

The invention further discloses a composition for use in the treatment of Parkinsonism, particularly PD, comprising torasemide and baclofen in a combination with a drug selected from acamprosate, sulfisoxazole, cinacalcet and mexiletine or salts, prodrugs, derivatives or sustained release formulations thereof, for combined, separate or sequential administration.

The invention further discloses a composition for use in the treatment of Parkinsonism, particularly PD, comprising torasemide and sulfisoxazole in a combination with a drug selected from acamprosate, baclofen, mexiletine and cinacalcet or salts, prodrugs, derivatives or sustained release formulations thereof, for combined, separate or sequential administration.

More particularly, the invention discloses compositions for use in the treatment of Parkinsonism, particularly PD, comprising at least one of the following drug combinations:
- baclofen and cinacalcet and mexiletine,
- cinacalcet and acamprosate and mexiletine,
- baclofen and acamprosate and cinacalcet,
- baclofen and acamprosate and torasemide,
- baclofen and acamprosate and mexiletine, or
- torasemide and baclofen and cinacalcet,
or salts, prodrugs, derivatives or sustained release formulations thereof, for combined, separate or sequential administration.

A further object of this invention resides in the use of a composition as defined above for the manufacture of a medicament for treating Parkinsonism, particularly PD.

As indicated previously, in a combination therapy of this invention, the compounds or drugs may be formulated together or separately, and administered together, separately or sequentially.

The invention also discloses a method of treating Parkinsonism, particularly PD, the method comprising simultaneously, separately or sequentially administering to a subject in need thereof an effective amount of a composition as disclosed above.

In this regard, the invention also discloses a method of treating Parkinsonism, particularly PD, the method comprising simultaneously, separately or sequentially administering to a subject in need thereof an effective amount of a drug combination as defined above.

The invention also discloses a method of treating Parkinsonism, particularly PD in a subject in need thereof, said method comprising administering simultaneously, separately or sequentially to the subject an effective amount of a composition of at least one compound selected from the group consisting of acamprosate, baclofen, cinacalcet, mexiletine, sulfisoxazole and torasemide.

The invention also discloses a method of treating Parkinsonism, particularly PD in a subject in need thereof, said method comprising administering simultaneously, separately or sequentially to the subject an effective amount of a combination of at least two compounds selected from the group consisting of acamprosate, baclofen, cinacalcet, mexiletine, sulfisoxazole and torasemide.

The invention also discloses a method of treating Parkinsonism, particularly PD in a subject in need thereof, said method comprising administering simultaneously, separately or sequentially to the subject an effective amount of a combination of at least one of the following drug combinations, or salts, prodrugs derivatives or sustained release formulation thereof:
- baclofen and acamprosate,
- baclofen and cinacalcet,
- cinacalcet and acamprosate,
- mexiletine and cinacalcet,
- torasemide and baclofen, or
- torasemide and sulfisoxazole.

The invention also discloses a method of treating Parkinsonism, particularly PD in a subject in need thereof, said method comprising administering simultaneously, separately or sequentially to the subject an effective amount of a combination of at least one of the following drug combinations, or salts, prodrugs, derivatives or sustained release formulation thereof:
- baclofen and cinacalcet and mexiletine,
- cinacalcet and acamprosate and mexiletine,
- baclofen and acamprosate and cinacalcet,
- baclofen and acamprosate and torasemide,
- baclofen and acamprosate and mexiletine, or
- torasemide and baclofen and cinacalcet.

The compositions of the invention typically comprise one or several pharmaceutically acceptable carriers or excipients. Also, for use in the present invention, the drugs or compounds are usually mixed with pharmaceutically acceptable excipients or carriers.

In this regard, a further object of this invention is a method of preparing a pharmaceutical composition, the method comprising mixing the above compounds or compound combinations in an appropriate excipient or carrier.

Although very effective *in vitro* and *in vivo,* depending on the subject or specific condition, the above methods, compositions or combination therapies may further be used in conjunction or association or combination with additional drugs or treatments.

Additional therapies used in conjunction with drug(s) or drug(s) combination(s) according to the present invention, may comprise one or more drug(s) that ameliorate symptoms of Parkinson's disease, one or more drug(s) that could be used for palliative treatment of Parkinson's disease or one or more drug(s) currently evaluated in the frame of clinical trials for treating Parkinson's disease.

Therefore, compositions of the invention can be combined with dopaminergic drugs such as dopamine precursors (preferably levodopa, melevodopa), dopamine receptor agonists (preferably talipexole, piribedil, rotigotine, bromocriptine, pergolide, cabergoline, lisuride, pramipexole, ropinirole or apomorphine) or inhibitors of dopamine-metabolizing enzymes (preferably selegiline, rasagiline).

Compositions of the invention can also be combined with other known treatments for PD, adjunctive treatments for PD, or treatment of the non-motor symptoms of PD or, preferably monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine or memantine.

Such use of compositions or combinations of the invention with the above mentioned therapies would permit the lowering of therapeutic doses of the concerned drugs and thus would reduce, delay or avoid known side effects associated with these drugs, for instance peak-dose dyskinesia which is observed in patients treated with levodopa.

In this regard, a further object of this invention relates to a composition, for use in the treatment of Parkinsonism, particularly PD, comprising a composition of the invention as defined above, in combination with at least one compound selected from the group consisting of levodopa, talipexole, piribedil, rotigotine, bromocriptine, pergolide, cabergoline, lisuride, pramipexole, ropinirole, apomorphine, selegiline, rasagiline, monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine and memantine, or salts or prodrugs or derivatives of any purity or sustained release formulations thereof.

Hence, this invention also discloses a composition for use in the treatment of Parkinsonism, particularly PD, comprising at least one compound selected from the group consisting of acamprosate, baclofen, cinacalcet, mexiletine, sulfisoxazole and torasemide or salt(s), prodrug(s), derivative(s) of any chemical purity, or sustained-release formulation(s) thereof, in combination with at least one compound selected from the group consisting of levodopa, talipexole, piribedil, rotigotine, bromocriptine, pergolide, cabergoline, lisuride, pramipexole, ropinirole, apomorphine, selegiline, rasagiline, monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine and memantine, or salts or prodrugs or derivatives of any purity or sustained release formulations thereof.

The invention also discloses a composition for use in the treatment of Parkinsonism, particularly PD comprising at least one of the following drug combinations:
- baclofen and levodopa,
- torasemide and levodopa,
- sulfisoxazole and levodopa,
- mexiletine and levodopa, or
- cinacalcet and levodopa,
or salts or prodrugs or derivatives of any purity or sustained release formulations thereof, for combined, separate or sequential administration.

The invention also discloses a composition *per se* comprising at least one of the following drug combinations:
- baclofen and levodopa,
- torasemide and levodopa,
- sulfisoxazole and levodopa,
- mexiletine and levodopa, or
- cinacalcet and levodopa,
or salts or prodrugs or derivatives of any purity or sustained release formulations thereof, for combined, separate or sequential administration.

The invention also discloses a method of treating Parkinsonism, particularly PD in a subject in need thereof, comprising administering simultaneously, separately or sequentially to the subject an effective amount of a combination of at least two compounds selected from the group consisting of acamprosate, baclofen, cinacalcet, mexiletine, sulfisoxazole and torasemide, in combination with at least one compound selected from the group consisting of levodopa, talipexole, piribedil, rotigotine, bromocriptine, pergolide, cabergoline, lisuride, pramipexole, ropinirole, apomorphine, selegiline, rasagiline, monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine and memantine, or salts or prodrugs or derivatives of any purity or sustained release formulations thereof.

In an embodiment, the invention relates to a composition for use in treating Parkinsonism, particularly PD, comprising an effective amount of baclofen and acamprosate or salts, or sustained release formulations thereof, in combination with at least one compound selected from the group consisting of levodopa, talipexole, piribedil, rotigotine, bromocriptine, pergolide, cabergoline, lisuride, pramipexole, ropinirole, apomorphine, selegiline, rasagiline, monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine and memantine, or salts or derivatives of any purity or sustained release formulations thereof.

The invention also discloses a method of treating Parkinsonism, particularly PD in a subject in need thereof, comprising administering simultaneously, separately or sequentially to the subject an effective amount of mexiletine and cinacalcet or salts, prodrugs, derivatives or sustained release formulations thereof, in combination with at least one compound selected from the group consisting of levodopa, talipexole, piribedil, rotigotine, bromocriptine, pergolide, cabergoline, lisuride, pramipexole, ropinirole, apomorphine, selegiline, rasagiline, monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine and memantine, or salts or prodrugs or derivatives of any purity or sustained release formulations thereof.

The invention discloses a method of treating Parkinsonism, particularly PD in a subject in need thereof, comprising administering simultaneously, separately or sequentially to the subject an effective amount of torasemide and baclofen or salts, prodrugs, derivatives or sustained release formulations thereof, in combination with at least one compound selected from the group consisting of levodopa talipexole, piribedil, rotigotine, bromocriptine, pergolide, cabergoline, lisuride, pramipexole, ropinirole, apomorphine, selegiline, rasagiline, monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine and memantine, or salts or prodrugs or derivatives of any purity or sustained release formulations thereof.

The invention discloses a method of treating Parkinsonism, particularly PD in a subject in need thereof, comprising administering simultaneously, separately or sequentially to the subject an effective amount of sulfisoxazole and torasemide or salts, prodrugs, derivatives or sustained release formulations thereof, in combination with at least one compound selected from the group consisting of levodopa, talipexole, piribedil, rotigotine, bromocriptine, pergolide, cabergoline, lisuride, pramipexole, ropinirole, apomorphine, selegiline, rasagiline, monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine and memantine, or salts or prodrugs or derivatives of any purity or sustained release formulations thereof.

The invention also discloses a method of treating Parkinsonism, particularly PD in a subject in need thereof, comprising administering simultaneously, separately or sequentially to the subject an effective amount of cinacalcet and acamprosate or salts, prodrugs, derivatives or sustained release formulations thereof, in combination with at least one compound selected from the group consisting of levodopa, talipexole, piribedil, rotigotine, bromocriptine, pergolide, cabergoline, lisuride, pramipexole, ropinirole, apomorphine, selegiline, rasagiline, monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine and memantine, or salts or prodrugs or derivatives of any purity or sustained release formulations thereof.

The invention also discloses a method of treating Parkinsonism, particularly PD in a subject in need thereof, comprising administering simultaneously, separately or sequentially to the subject an effective amount of baclofen and cinacalcet or salts, prodrugs, derivatives or sustained release formulations thereof, in combination with at least one compound selected from the group consisting of levodopa, talipexole, piribedil, rotigotine, bromocriptine, pergolide, cabergoline, lisuride, pramipexole, ropinirole, apomorphine, selegiline, rasagiline, monosialotetrahexosylganglioside, citicoline, droxidopa mazaticol, promethazine, quetiapine, procyclidine, orphenadrine, domperidone, benzatropine, trihexyphenidyl, biperiden, clozapine, desipramine, citalopram, nortriptyline, paroxetine, atomoxetine, venlafaxine, amantadine, donepezil, rivastigmine and memantine, or salts or prodrugs or derivatives of any purity or sustained release formulations thereof.

The preferred compositions disclosed in this invention, for use in the treatment of Parkinsonism, particularly PD, comprise at least one of the following drug combinations, or salts, prodrugs, derivatives or sustained release formulations thereof, the drugs in each of said combinations being for combined, separate or sequential administration:
- baclofen and acamprosate and levodopa,
- mexiletine and cinacalcet and levodopa,
- torasemide and baclofen and levodopa,
- baclofen and cinacalcet and levodopa,
- cinacalcet and acamprosate and levodopa, or
- sulfisoxazole and torasemide and levodopa.

The invention also discloses a composition *per se* comprising at least one of the following drug combinations, or salts, prodrugs, derivatives or sustained release formulations thereof, the drugs in each of said combinations being for simultaneous, separate or sequential administration:
- baclofen and acamprosate and levodopa,
- mexiletine and cinacalcet and levodopa,
- torasemide and baclofen and levodopa,
- baclofen and cinacalcet and levodopa,
- cinacalcet and acamprosate and levodopa, or
- sulfisoxazole and torasemide and levodopa.

The invention also discloses compositions, for use in the treatment of Parkinsonism, particularly PD, comprising at least one of the following drug combinations, or salts, prodrugs, derivatives or sustained release formulations thereof, the drugs in each of said combinations being for combined, separate or sequential administration:
- baclofen, cinacalcet, mexiletine and levodopa,
- cinacalcet, acamprosate, mexiletine and levodopa,
- baclofen, acamprosate, cinacalcet and levodopa,
- baclofen, acamprosate, torasemide and levodopa,
- baclofen, acamprosate, mexiletine, and levodopa, or
- torasemide, baclofen, cinacalcet and levodopa.

In a particular embodiment, when compositions or combination therapies of the invention comprise dopamine precursor, they can be further combined with at least one compound selected from peripheral dopa decarboxylase inhibitors, catechol-O-methyl transferase inhibitors or monoamine oxidase inhibitors. More particularly, when compositions or combination therapies of the invention comprise a dopamine precursor, they can be further combined with at least one compound selected from carbidopa, benserazide, tolcapone, entacapone, selegiline or rasagiline.

A further object of this invention resides in the use of a composition as defined above for the manufacture of a medicament for treating Parkinsonism, particularly PD.

In another embodiment, compositions or combination therapies of the invention can be used in conjunction with surgical therapy for Parkinson's disease such as deep brain stimulation. More particularly, surgical therapies are deep brain stimulation of the subthalamic nucleus or of the *globus pallidus interna.*

In this regard, the invention also discloses a composition comprising at least one compound selected from the group consisting of acamprosate, baclofen, cinacalcet, mexiletine, sulfisoxazole and torasemide, or salt(s), prodrug(s), derivative(s) of any chemical purity, or sustained-release formulation(s) thereof, for use in combination with deep brain stimulation of the subthalamic nucleus or of the *globus pallidus interna,* in the treatment of Parkinsonism, particularly PD.

PD motor symptoms can develop lately when the dopaminergic denervation of the striatum and lose of *substantia nigra* dopaminergic neurons are already widely occurring. Thus, the treatment of PD before motor symptoms appearance and in prevention is essential in order to alter the progression and course of the disease.

In this regard, in a preferred embodiment, any of the above compositions or combination therapies can be used for the prevention, prophylaxis or retardation of symptoms provoked by or of the causes of Parkinson's disease.

The combination of early detection of non-motor symptoms, most particularly anosmia, with imaging techniques (Single-photon emission computed technology, Positron Emission Tomography) to assess changes in striatal dopamine transporter may be a suitable approach to identify at risk PD patients prior to the appearance of motor symptoms, thus allowing early start of neuroprotective therapy.

Some PD cases can be attributed to mutations within genes such as SNCA (alpha-synuclein), PRKN (parkin), LRRK2 (leucine-rich repeat kinase 2), PINK1 (PTEN-induced putative kinase 1), DJ-1 and ATP13A2 and eleven gene loci (PARK1-PARK11). In this regard, in a particular embodiment, the invention relates to the use of the above compositions or combination therapies for the treatment of PD in a subject having a mutation in at least one of the following genes: SNCA, PRKN, LRRK2, PINK1, DJ-1, ATP13A2 and PARK1 to PARK11.

High concentrations exposure or chronic exposure to metals such as manganese, copper or lead, or chemicals, such as pesticides (*e.g*. paraquat, rotenone and maneb), are likely to cause Parkinsonism, particularly PD. In this regard, in a particular embodiment, the invention relates to the use of the above compositions or combination therapies in the treatment of Parkinsonism, particularly PD, in a subject exposed, suspected to have been exposed or at risk of being exposed to chemicals or metals known to be risk factors for developing PD or related disorders.

In a preferred embodiment, the above compositions or combination therapies can be used in a subject who is at risk of developing Parkinson's disease or symptoms associated with Parkinson's disease.

Therapy according to the invention may be provided at home, the doctor's office, a clinic, a hospital's outpatient department, or a hospital, so that the doctor can observe the therapy's effects closely and make any adjustments that are needed.

The duration of the therapy depends on the stage of the disease being treated, age and condition of the patient, and how the patient responds to the treatment. The dosage, frequency and mode of administration of each component of the combination can be controlled independently. For example, one drug may be administered orally while the second drug may be administered intramuscularly. Combination therapy may be given in on-and-off cycles that include rest periods so that the patient's body has a chance to recovery from any as yet unforeseen side-effects. The drugs may also be formulated together such that one administration delivers all drugs.

The administration of each drug of the combination may be by any suitable means that results in a concentration of the drug that, combined with the other component, is able to ameliorate the patient condition or efficiently treat the disease or disorder.

While it is possible for the drugs of the combination to be administered as the pure chemical, it is preferable to present them as a pharmaceutical composition, also referred to in this context as pharmaceutical formulation. Possible compositions include those suitable for oral, rectal, topical (including transdermal, buccal and sublingual), or parenteral (including subcutaneous, intramuscular, intravenous and intradermal) administration.

More commonly these pharmaceutical formulations are prescribed to the patient in "patient packs" containing a number dosing units or other means for administration of metered unit doses for use during a distinct treatment period in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve patient compliance with the physician's instructions. Thus, the invention further includes a pharmaceutical formulation, as herein before described, in combination with packaging material suitable for said formulations. In such a patient pack the intended use of a formulation for the combination treatment can be inferred by instructions, facilities, provisions, adaptations and/or other means to help using the formulation most suitably for the treatment. Such measures make a patient pack specifically suitable for and adapted for use for treatment with the combination of the present invention.

The drug may be contained, in any appropriate amount, in any suitable carrier substance. The drug may be present in an amount of up to 99% by weight of the total weight of the composition. The composition may be provided in a dosage form that is suitable for the oral, parenteral (*e.g*., intravenously, intramuscularly), rectal, cutaneous, nasal, vaginal, inhalant, skin (patch), or ocular administration route. Thus, the composition may be in the form of, *e.g*., tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, or aerosols.

The pharmaceutical compositions may be formulated according to conventional pharmaceutical practice (see, *e.g*., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York).

Pharmaceutical compositions according to the invention may be formulated to release the active drug substantially immediately upon administration or at any predetermined time or time period after administration.

The controlled release formulations include (i) formulations that create a substantially constant concentration of the drug within the body over an extended period of time; (ii) formulations that after a predetermined lag time create a substantially constant concentration of the drug within the body over an extended period of time; (iii) formulations that sustain drug action during a predetermined time period by maintaining a relatively, constant, effective drug level in the body with concomitant minimization of undesirable side effects associated with fluctuations in the plasma level of the active drug substance; (iv) formulations that localize drug action by, *e.g*., spatial placement of a controlled release composition adjacent to or in the diseased tissue or organ; and (v) formulations that target drug action by using carriers or chemical derivatives to deliver the drug to a particular target cell type.

Administration of drugs in the form of a controlled release formulation is especially preferred in cases in which the drug has (i) a narrow therapeutic index (*i.e*., the difference between the plasma concentration leading to harmful side effects or toxic reactions and the plasma concentration leading to a therapeutic effect is small; in general, the therapeutic index, TI, is defined as the ratio of median lethal dose (LD50) to median effective dose (ED50)); (ii) a narrow absorption window in the gastrointestinal tract; or (iii) a very short biological half-life so that frequent dosing during a day is required in order to sustain the plasma level at a therapeutic level.

Any of a number of strategies can be pursued in order to obtain controlled release in which the rate of release outweighs the rate of metabolism of the drug in question. Controlled release may be obtained by appropriate selection of various formulation parameters and ingredients, including, *e.g*., various types of controlled release compositions and coatings. Thus, the drug is formulated with appropriate excipients into a pharmaceutical composition that, upon administration, releases the drug in a controlled manner (single or multiple unit tablet or capsule compositions, oil solutions, suspensions, emulsions, microcapsules, microspheres, nanoparticles, patches, and liposomes).

### Solid Dosage Forms for Oral Use

Formulations for oral use include tablets containing the composition of the invention in a mixture with non-toxic pharmaceutically acceptable excipients. These excipients may be, for example, inert diluents or fillers (*e.g*., sucrose, microcrystalline cellulose, starches including potato starch, calcium carbonate, sodium chloride, calcium phosphate, calcium sulfate, or sodium phosphate); granulating and disintegrating agents (*e.g*., cellulose derivatives including microcrystalline cellulose, starches including potato starch, croscarmellose sodium, alginates, or alginic acid); binding agents (*e.g*., acacia, alginic acid, sodium alginate, gelatin, starch, pregelatinized starch, microcrystalline cellulose, carboxymethylcellulose sodium, methylcellulose, hydroxypropyl methylcellulose, ethylcellulose, polyvinylpyrrolidone, or polyethylene glycol); and lubricating agents, glidants, and antiadhesives (*e.g*., stearic acid, silicas, or talc). Other pharmaceutically acceptable excipients can be colorants, flavoring agents, plasticizers, humectants, buffering agents, and the like.

The tablets may be uncoated or they may be coated by known techniques, optionally to delay disintegration and absorption in the gastrointestinal tract and thereby providing a sustained action over a longer period. The coating may be adapted to release the active drug substance in a predetermined pattern (*e.g*., in order to achieve a controlled release formulation) or it may be adapted not to release the active drug substance until after passage of the stomach (enteric coating). The coating may be a sugar coating, a film coating (*e.g*., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or an enteric coating (*e.g*., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). A time delay material such as, *e.g*., glyceryl monostearate or glyceryl distearate may be employed.

The solid tablet compositions may include a coating adapted to protect the composition from unwanted chemical changes, (*e.g*., chemical degradation prior to the release of the active drug substance). The coating may be applied on the solid dosage form in a similar manner as that described in Encyclopedia of Pharmaceutical Technology.

Drugs may be mixed together in the tablet, or may be partitioned. For example, a first drug is contained on the inside of the tablet, and a second drug is on the outside, such that a substantial portion of the second drug is released prior to the release of the first drug.

Formulations for oral use may also be presented as chewable tablets, or as hard gelatin capsules wherein the active ingredient is mixed with an inert solid diluent (*e.g*., potato starch, microcrystalline cellulose, calcium carbonate, calcium phosphate or kaolin), or as soft gelatin capsules wherein the active ingredient is mixed with water or an oil medium, for example, liquid paraffin, or olive oil. Powders and granulates may be prepared using the ingredients mentioned above under tablets and capsules in a conventional manner.

Controlled release compositions for oral use may, *e.g*., be constructed to release the active drug by controlling the dissolution and/or the diffusion of the active drug substance.

Dissolution or diffusion controlled release can be achieved by appropriate coating of a tablet, capsule, pellet, or granulate formulation of drugs, or by incorporating the drug into an appropriate matrix. A controlled release coating may include one or more of the coating substances mentioned above and/or, *e.g*., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, *e.g*., hydrated metylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition containing one or more of the drugs of the claimed combinations may also be in the form of a buoyant tablet or capsule (*i.e*., a tablet or capsule that, upon oral administration, floats on top of the gastric content for a certain period of time). A buoyant tablet formulation of the drug(s) can be prepared by granulating a mixture of the drug(s) with excipients and 20-75% w/w of hydrocolloids, such as hydroxyethylcellulose, hydroxypropylcellulose, or hydroxypropylmethylcellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice.

### Liquids for Oral Administration

Powders, dispersible powders, or granules suitable for preparation of an aqueous suspension by addition of water are convenient dosage forms for oral administration. Formulation as a suspension provides the active ingredient in a mixture with a dispersing or wetting agent, suspending agent, and one or more preservatives. Suitable suspending agents are, for example, sodium carboxymethylcellulose, methylcellulose, sodium alginate, and the like.

### Parenteral Compositions

The pharmaceutical composition may also be administered parenterally by injection, infusion or implantation (intravenous, intramuscular, subcutaneous, or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation.

Compositions for parenteral use may be provided in unit dosage forms (*e.g*., in single-dose ampoules), or in vials containing several doses and in which a suitable preservative may be added (see below). The composition may be in form of a solution, a suspension, an emulsion, an infusion device, or a delivery device for implantation or it may be presented as a dry powder to be reconstituted with water or another suitable vehicle before use. Apart from the active drug(s), the composition may include suitable parenterally acceptable carriers and/or excipients. The active drug(s) may be incorporated into microspheres, microcapsules, nanoparticles, liposomes, or the like for controlled release. The composition may include suspending, solubilizing, stabilizing, pH-adjusting agents, and/or dispersing agents.

The pharmaceutical compositions according to the invention may be in the form suitable for sterile injection. To prepare such a composition, the suitable active drug(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, and isotonic sodium chloride solution. The aqueous formulation may also contain one or more preservatives (*e.g*., methyl, ethyl or n-propyl p-hydroxybenzoate). In cases where one of the drugs is only sparingly or slightly soluble in water, a dissolution enhancing or solubilizing agent can be added, or the solvent may include 10-60% w/w of propylene glycol or the like.

Controlled release parenteral compositions may be in form of aqueous suspensions, microspheres, microcapsules, magnetic microspheres, oil solutions, oil suspensions, or emulsions. Alternatively, the active drug(s) may be incorporated in biocompatible carriers, liposomes, nanoparticles, implants, or infusion devices. Materials for use in the preparation of microspheres and/or microcapsules are, *e.g*., biodegradable/bioerodible polymers such as polygalactin, poly-(isobutyl cyanoacrylate), poly(2-hydroxyethyl-L-glutamnine). Biocompatible carriers that may be used when formulating a controlled release parenteral formulation are carbohydrates (*e.g*., dextrans), proteins (*e.g*., albumin), lipoproteins, or antibodies. Materials for use in implants can be non-biodegradable (*e.g*., polydimethyl siloxane) or biodegradable (*e.g*., poly(caprolactone), poly(glycolic acid) or poly(ortho esters)).

### Alternative routes

Although less preferred and less convenient, other administration routes, and therefore other formulations, may be contemplated. In this regard, for rectal application, suitable dosage forms for a composition include suppositories (emulsion or suspension type), and rectal gelatin capsules (solutions or suspensions). In a typical suppository formulation, the active drug(s) are combined with an appropriate pharmaceutically acceptable suppository base such as cocoa butter, esterified fatty acids, glycerinated gelatin, and various water-soluble or dispersible bases like polyethylene glycols. Various additives, enhancers, or surfactants may be incorporated.

The pharmaceutical compositions may also be administered topically on the skin for percutaneous absorption in dosage forms or formulations containing conventionally non-toxic pharmaceutical acceptable carriers and excipients including microspheres and liposomes. The formulations include creams, ointments, lotions, liniments, gels, hydrogels, solutions, suspensions, sticks, sprays, pastes, plasters, and other kinds of transdermal drug delivery systems. The pharmaceutically acceptable carriers or excipients may include emulsifying agents, antioxidants, buffering agents, preservatives, humectants, penetration enhancers, chelating agents, gel-forming agents, ointment bases, perfumes, and skin protective agents.

The preservatives, humectants, penetration enhancers may be parabens, such as methyl or propyl p-hydroxybenzoate, and benzalkonium chloride, glycerin, propylene glycol, urea, etc.

The pharmaceutical compositions described above for topical administration on the skin may also be used in connection with topical administration onto or close to the part of the body that is to be treated. The compositions may be adapted for direct application or for application by means of special drug delivery devices such as dressings or alternatively plasters, pads, sponges, strips, or other forms of suitable flexible material.

### Dosages and duration of the treatment

It will be appreciated that the drugs of the combination may be administered concomitantly, either in the same or different pharmaceutical formulation or sequentially. If there is sequential administration, the delay in administering the second (or additional) active ingredient should not be such as to lose the benefit of the efficacious effect of the combination of the active ingredients. A minimum requirement for a combination according to this description is that the combination should be intended for combined use with the benefit of the efficacious effect of the combination of the active ingredients. The intended use of a combination can be inferred by facilities, provisions, adaptations and/or other means to help using the combination according to the invention.

Therapeutically effective amounts of the drugs in a combination of this invention include, *e.g*., amounts that are effective for reducing Parkinson's disease symptoms, halting or slowing the progression of the disease once it has become clinically manifest, or prevention or reduction of the risk of developing the disease.

Although the active drugs of the present invention may be administered in divided doses, for example two or three times daily, a single daily dose of each drug in the combination is preferred, with a single daily dose of all drugs in a single pharmaceutical composition (unit dosage form) being most preferred.

Administration can be one to several times daily for several days to several years, and may even be for the life of the patient. Chronic or at least periodically repeated long-term administration is indicated in most cases.

The term "unit dosage form" refers to physically discrete units (such as capsules, tablets, or loaded syringe cylinders) suitable as unitary dosages for human subjects, each unit containing a predetermined quantity of active material or materials calculated to produce the desired therapeutic effect, in association with the required pharmaceutical carrier.

The amount of each drug in a preferred unit dosage composition depends upon several factors including the administration method, the body weight and the age of the patient, the stage of the disease, the risk of potential side effects considering the general health status of the person to be treated. Additionally, pharmacogenomic (the effect of genotype on the pharmacokinetic, pharmacodynamic or efficacy profile of a therapeutic) information about a particular patient may affect the dosage used.

Except when responding to especially impairing cases, where higher dosages may be required, the preferred dosage of each drug in the combination will usually lie within the range of doses not above the dosage usually prescribed for long-term maintenance treatment or proven to be safe in phase 3 clinical studies.

One remarkable advantage of the invention is that each compound may be used at low doses in a combination therapy, while producing, in combination, a substantial clinical benefit to the patient. The combination therapy may indeed be effective at doses where the compounds have individually low or no effect. Accordingly, a particular advantage of the invention lies in the ability to use sub-optimal doses of each compound, *i.e*., doses which are lower than therapeutic doses usually prescribed, preferably 1/2 of therapeutic doses, more preferably 1/3, 1/4, 1/5, or even more preferably 1/10 of therapeutic doses. In particular examples, doses as low as 1/20, 1/30, 1/50, 1/100, or even lower, of therapeutic doses are used.

At such sub-therapeutic dosages, the compounds would exhibit no side effect, while the combination(s) according to the invention are fully effective in treating Parkinson's disease.

A preferred dosage corresponds to amounts from 1% up to 50% of those usually prescribed for long-term maintenance treatment.

The most preferred dosage may correspond to amounts from 1% up to 10% of those usually prescribed for long-term maintenance treatment.

Specific examples of dosages of drugs (Quantity equivalent to active molecule) for use as disclosed in the invention are provided below:
- acamprosate: 1000 mg or less per day, preferably less than 500 mg per day, preferably less than 400 mg per day, more preferably less than 200 mg per day, more preferably less than 50 mg per day, even more preferably from about 1 to 50 mg per day, or even less that 10 mg per day, such dosages being particularly suitable for oral administration.
- baclofen: 150 mg or less per day, preferably less than 100 mg per day, more preferably less than 50 mg per day, more preferably less than 30 mg per day, even more preferably between 0.01 mg to 30 mg per day, such dosages being particularly suitable for oral administration.
- cinacalcet: 150 mg or less per day, preferably less than 100 mg per day, preferably less than 50 mg per day, more preferably less than 36 mg per day, and even more preferably between 0.01 to 25 mg per day, such dosages being particularly suitable for oral administration,

- mexiletine: 120 mg or less per day, preferably less than 60 mg per day, more preferably less than 30 mg per day, more preferably less than 15 mg per day, even more preferably between 6 to 15 mg per day, such dosages being particularly suitable for oral administration,
- torasemide: 4 mg or less per day, preferably less than 2 mg per day, more preferably less than 1 mg per day, more preferably less than 0.5 mg per day, and even more preferably between 0.05 to 0.5 mg per day, such dosages being particularly suitable for oral administration,
- sulfisoxazole 800 mg or less per day, preferably less than 400mg, more preferably less than 200 mg per day, more preferably less than 100mg per day, even more preferably less than 20 mg per day, such dosages being particularly suitable for oral administration,
- levodopa 1,5 g or less per day, preferably less than 750 mg per day, more preferably less than 375 mg per day, even more preferably less than 100 mg per day, such dosages being particularly suitable for oral administration.

It will be understood that the amount of the drug or the drug combination actually administered will be determined by a physician, in the light of the relevant circumstances including the condition or conditions to be treated, the exact composition to be administered, the age, weight, and response of the individual patient, the severity of the patient's symptoms, and the chosen route of administration. Therefore, the above dosage ranges are intended to provide general guidance and support for the teachings herein, but are not intended to limit the scope of the invention. Some of the following examples are mentioned for comparison purposes only and do not form part of the present invention.

### EXAMPLES

*All animal procedures have been conducted in compliance to the National Institute of Health (NIH) guidelines for the care and use of laboratory animals, and approved by the National Animal Experiment Board.*

### A- PREVENTION OF GLUTAMATE TOXICITY ON NEURONAL CELLS

Glutamate toxicity is involved in the pathogenesis of Parkinson's disease. In this set of experiment, candidate compounds have been tested for their ability to prevent or reduce the toxic effects of glutamate toxicity on neuronal cells. The drugs are first tested individually, followed by assays of their combinatorial action.

### Neuronal cell preparation

The efficacy of drug combinations of the invention is assessed on primary cortical neuron cells.

Rat cortical neurons were cultured as described by Singer *et al.* (30). Briefly pregnant female rats of 15 days gestation were killed by cervical dislocation (Rats Wistar) and the foetuses were removed from the uterus. The cortex was removed and placed in ice-cold medium of Leibovitz (L15) containing 2% of Penicillin 10.000 U/ml and Streptomycin 10mg/ml and 1% of bovine serum albumin (BSA). Cortices were dissociated by trypsin for 20 min at 37°C (0.05%). The reaction was stopped by the addition of Dulbecco's modified Eagle's medium (DMEM) containing DNase1 grade II and 10% of foetal calf serum (FCS). Cells were then mechanically dissociated by 3 serial passages through a 10 ml pipette and centrifuged at 515 x g for 10 min at +4°C. The supernatant was discarded and the pellet of cells was re-suspended in a defined culture medium consisting of Neurobasal supplemented with B27 (2%), L-glutamine (0.2mM), 2% of PS solution and 10 ng/ml of BDNF. Viable cells were counted in a Neubauer cytometer using the trypan blue exclusion test. The cells were seeded at a density of 30 000 cells/well in 96 well-plates (wells were pre-coated with poly-L-lysine (10 µg/ml)) and were cultured at +37°C in a humidified air (95%)/CO2 (5%) atmosphere.

### Glutamate toxicity assays

The neuroprotective effect of compounds is assessed by quantification of the neurite network (Neurofilament immunostaining (NF)) which specifically reveals the glutamatergic neurons.

After 12 days of neuron culture, drugs of the candidate combinations are solved in culture medium (+0.1% DMSO). Candidate combinations are then pre-incubated with neurons for 1 hour before the glutamate injury. One hour after incubation with, glutamate is added for 20 min, to a final concentration of 40µM, in presence of candidate combinations, in order to avoid further drug dilutions. At the end of the incubation, medium is changed with medium with candidate combination but without glutamate. The culture is fixed 24 hours after glutamate injury. MK801 (dizocilpinehydrogen maleate, 77086-22-7 - 20 µM) is used as a positive control.

After permeabilization with saponin (Sigma), cells are blocked for 2h with PBS containing 10% goat serum, then the cells are incubated with mouse monoclonal primary antibody against Neurofilament antibody (NF, Sigma). This antibody is revealed with Alexa Fluor 488 goat anti-mouse IgG.

Nuclei of cells are labeled by a fluorescent marker (Hoechst solution, SIGMA), and neurite network quantified. Six wells per condition are used to assess neuronal survival in 3 different cultures.

### Results

All of the tested drug combinations give a protective effect against glutamate toxicity for cortical neuronal cells. Results are shown in Table 2 below.

As exemplified in figures 1 to 3, combinations of the invention strongly protect neurons from glutamate toxicity under experimental conditions described above. It is noteworthy that an effective protection is noticed using drug concentrations at which drugs used alone have no significant or lower protective effect.

Indeed, as exemplified in figure 1, mexiletine - cinacalcet combination efficiently protects neuronal cells from glutamate toxicity, whereas no protection is afforded by the single drugs. baclofen - acamprosate (figure 3) combination gives a protective effect against glutamate toxicity for cortical neuronal cells. Combination of baclofen and acamprosate induce an improvement of more than 200% compared to acamprosate alone and more than 47% compared to baclofen used alone.

**Table 2**

| **Drug Combination** | **Neuroprotective effect against glutamate toxicity** |
|---|---|
| baclofen and torasemide | + |
| baclofen-acamprosate-torasemide | + |
| mexiletine and cinacalcet | + |
| sulfisoxazole and torasemide | + |
| baclofen and acamprosate | + |
| acamprosate and cinacalcet | + |
| baclofen and cinacalcet | + |

### B- PROTECTIVE EFFECT AGAINST ISCHEMIA/HYPOXIA INDUCED NEURONAL CELL DEATH.

### Rat neuronal cortical cells preparation

Cells are prepared as previously.

### Oxygen and glucose deprivation assays (in vitro model of ischemia)

The neuroprotective effect of compounds is assessed by quantification of the neurite network (Neurofilament immunostaining (NF)) using MAP2 antibody. Riluzole, a neuroprotective drug, (Riluteck^{®}, 5 µM) is used as positive control.

After 10 days of neuron culture, candidate drugs are solved in culture medium (+0.1% DMSO) and then pre-incubated with neurons for 1 hour before the oxygen and glucose deprivation. One hour after candidate drug incubation, the medium is removed and fresh medium without glucose is added. This medium is composed by DMEM without glucose (Invitrogen) supplemented with 2% B27, 0.2 mM L-glutamine, 1% PS solution, 10 ng/ml of BDNF. The cells are transferred into an anaerobic incubator with 95 % N₂ and 5 % CO₂ at 37°C.

After 2 hours, 25 mM of D-Glucose will be added in culture medium and cells are transferred in classic incubator with 95% air /5% CO₂ at 37°C. After 24 hours of oxygen glucose reperfusion, cells are fixed by a cold solution of alcool/acetic acid during 5 minutes.

After permeabilization with saponin (Sigma), cells are blocked for 2 hours with PBS containing 10% goat serum, then the cells are incubated with mouse monoclonal primary antibody against MAP2 (MAP2, Sigma). These antibodies are revealed with Alexa Fluor 488 goat anti-mouse IgG (Molecular probe).

Nuclei of cells are labelled by a fluorescent marker (Hoechst solution, SIGMA). Six wells per condition are used to assess neuronal survival in 3 different cultures.

Six wells per condition are used to assess neuronal survival in 3 different cultures. For each condition 2x10 pictures per well are taken and analyzed using InCell Analyzer TM 1000 (GE Healthcare) with 20x magnification.

### Results

As shown in Table 3 below, the claimed drug combinations give a protective effect against ischemia/hypoxia induced cell death for cortical neuronal cells.

**Table 3**

| **Drug Combination** | **Protective effect against ischemia/hypoxia** |
|---|---|
| baclofen and torasemide | + |
| Baclofen - acamprosate - torasemide | + |
| mexiletine and cinacalcet | + |
| sulfisoxazole and torasemide | + |
| baclofen and acamprosate | + |
| acamprosate and cinacalcet | + |
| baclofen and cinacalcet | + |

Figures 4-6 further show that the combination treatments claimed and/or disclosed in the invention strongly protect neurons from oxygen and glucose deprivation. As shown in figures 4-6, an effective protection is observed using drug concentrations at which drugs, alone, have no significant protective effect. For example, a significant protective effect of baclofen (80 nM) /acamprosate (0.32 nM) combination or cinacalcet (64 pM) /mexiletine (25.6 pM) combination or torasemide (80 nM) /sulfisoxazole (1.36 nM), in ischemia is observed, while no significant protection is obtained when baclofen, acamprosate, cinacalcet, mexiletine, torasemide and sulfisoxazole are used alone, at the same concentrations.

These results therefore demonstrate a potent and synergistic effect of the combination therapies on oxidative stress and mitochondrial dysfunction or apoptosis which are underlie under ischemic conditions as well as Parkinson's disease.

### C- NEURO-PROTECTIVE EFFECT OF DRUGS AGAINST 6OHDA INJURY ON DOPAMINERGIC NEURONS

6-hydroxydopamine (6-OHDA) is a neurotoxic drug which selectively destroys dopaminergic neurons by generating reactive oxygen species in the cells. 6OHDA toxicity is commonly used *in vitro* and *in vivo* to study Parkinsonism.

### Culture of mesencephalic dopaminergic neurons

Rat dopaminergic neurons were cultured as described by Schinelli *et al.* (31). Pregnant female rats of 15 days gestation were killed by cervical dislocation (Rats Wistar; Janvier) and the foetuses removed from the uterus. The embryonic midbrains were removed and placed in ice-cold medium of Leibovitz (L15; PanBiotech) containing 2% of Penicillin-Streptomycin (PS; PanBiotech) and 1% of bovine serum albumin (BSA; PanBiotech). Only the ventral portions of the mesencephalic flexure were used for the cell preparations as this is the region of the developing brain rich in dopaminergic neurons. The midbrains were dissociated by trypsinisation for 20 min at 37°C (Trypsin EDTA IX; PanBiotech). The reaction is stopped by the addition of Dulbecco's modified Eagle's medium (DMEM; PanBiotech) containing DNAase I grade II (0.1 mg/ml; PanBiotech) and 10% of foetal calf serum (FCS; Invitrogen). Cells were then mechanically dissociated by 3 passages through a 10 ml pipette and centrifuged at 180 x g for 10 min at +4°c on a layer of BSA (3.5%) in L15 medium. The supernatant was discarded and the cells of pellet were re-suspended in a defined culture medium consisting of Neurobasal (Invitrogen) supplemented with B27 (2%; Invitrogen), L-glutamine (2 mM; PanBiotech) and 2% of PS solution and 10 ng/ml of Brain-derived neurotrophic factor (BDNF, PanBiotech) and 1 ng/ml of Glial-Derived Neurotrophic Factor (GDNF, PanBiotech). Viable cells were counted in a Neubauer cytometer using the trypan blue exclusion test. The cells were seeded at a density of 40 000 cells/well in 96 well-plates (pre-coated with poly-L-lysine (Greiner)) and are cultured at 37°C in a humidified air (95%)/CO2 (5%) atmosphere. Half of the medium was changed every 2 days with fresh medium. Five to six percents of the neuronal cell population were dopaminergic neurons.

### 6-OHDA and tests compounds exposure

On day 6 of culture, the medium was removed and fresh medium was added, without or with 6OHDA at the following concentrations: 20 µM during 48 hours diluted in control medium. Test compounds were pre-incubated for 1h before the 6-OHDA application during 48 hours.

### End point evaluation: measure of total number of THpositive neurons

After 48 hours of intoxication with 6OHDA, cells were fixed by a solution of 4% paraformaldehyde (Sigma) in PBS, pH = 7.3 for 20 min at room temperature. The cells were washed again twice in PBS, and then permeabilized and non-specific sites were blocked with a solution of PBS containing 0.1% of saponin (Sigma) and 1% FCS for 15 min at room temperature. Then, cells were incubated with Monoclonal Anti-Tyrosine Hydroxylase antibody produced in mouse (TH, Sigma) at dilution of 1/1000 in PBS containing 1% FCS, 0.1% saponin, for 2 h at room temperature. These antibodies were revealed with Alexa Fluor 488 goat anti-mouse IgG (Molecular Probes) at the dilution 1/800 in PBS containing 1% FCS, 0.1% saponin, for 1 h at room temperature.

For each condition, 2 x 10 pictures (representing ∼ 80 % of total well area) per well were taken using InCell AnalyzerTM 1000 (GE Healthcare) with 10x magnification. All images were taken in the same conditions. Analysis of the number of TH positive neurons were done using Developer software (GE Healthcare).

Data are expressed in percentage of control conditions (no intoxication, no 6OHDA = 100%) in order to express the 6OHDA injury. All values are expressed as mean +/- SEM (s.e.mean) of the 3 cultures (n = 6 wells per condition per culture). Statistical analyses consist in an ANOVA followed by the Dunnett's and PLSD Fisher's tests when it was allowed using Statview software version 5.0.

### Results

A neuroprotective effect is observed, for the claimed combinations of the invention, in TH neurons survival test after 48 h 6-OHDA injury on dopaminergic neurons.

A 48h 6-OHDA (20 µM) incubation with mesencephalic neurons produced a significant intoxication of dopaminergic neurons (around -33% of TH neurons) in all the experiments (control, figures 7-9).

BDNF was used as a positive control. One hour of BDNF pre-treatment at 1,85 nM significantly protected the dopaminergic neurons from this 6-OHDA injury.

As shown in table 4 below, the claimed drug combinations give a protective effect against 6OHDA injury in dopaminergic neuronal cells.

As shown in figures 7-9, baclofen-acamprosate, baclofen-torasemide, mexiletine-cinacalcet successfully protect dopaminergic neurons from 6-OHDA intoxication, in a dose dependent manner.

**Table 4**

| **Drug Combination** | **Protective effect against 6OHDA induced stereotaxic akinesia** |
|---|---|
| baclofen and torasemide | + |
| baclofen-acamprosate-torasemide | + |
| mexiletine and cinacalcet | + |
| sulfisoxazole and torasemide | + |
| baclofen and acamprosate | + |
| acamprosate and cinacalcet | + |
| baclofen and cinacalcet | + |

### D- EFFECTS ON DOPAMINERGIC NEURONAL LOSS IN VIVO AND ON MOTOR SYMPTOMS

### Animal husbandry and surgical procedure

Wistar rats (5 weeks) were used after an acclimatization period of at least 5 days. Surgery was performed under ketamine (50 mg/kg) and xylazine (10 mg/kg). Animals received an unilateral injection of 12 µg of 6-OHDA (sigma Aldrich) dissolved in 6 µl of 0.9% sterile NaCl containing 0.1% ascorbic acid (to protect 6-OHDA from oxidation), at the flow rate of 1 µl/min, in the left *substantia nigra pars compacta.* The stereotaxic coordinates of the injection site will be: anteroposterior + 2.2 mm, lateral 2.0 mm, dorsoventral + 3 mm with the incisor bar at + 5.0 mm above the interaural plane, according to the rat stereotaxic atlas by De Groot (1959) (32).

### Drug(s) treatment

The first administration of treatment (or vehicle) has been performed the day before the stereotaxic injection of 6-OHDA (for the lesioned groups) or vehicle and all along the 15 days preceding the behavioral tests. During the study and for each animal, the volume of P.O. administrations was determined on the basis of the mean body weight of the animals of the corresponding group. Body weights will be determined twice a week and the volume of administration will thus be adjusted consequently.

Vehicles and compounds have been administered twice a day through the P.O. route, in the morning and in the afternoon; eight hours (+/- 30 min) separated the two administrations around 9:30 AM and around 17:30 PM.

On the day of behavioral tests, the drugs have been administered around 1 h for L-DOPA treated group and around 2 h (+/- 15 min) for the drug combinations before the behavioral test, for each animal.

### Behavioural testing

Each test was performed before surgery (2 or 3 days before) to determine the basal level value. Assessment of behavioral functions is performed 15 days after surgical injection of 6-OHDA using the two different tests.

Initiation time test: The animal was hold by a trained technician in front of a plane surface. Only one of the two forelimbs was left free to move. The time that was necessary to initiate the movement toward the plane surface has been recorded using 180 sec as break-off point (33).

Stepping test : The rat was hold by the experimenter and only one of the two forelimbs have been left free to move above a plane surface. The other hand fixed the forelimb not to be monitored with one paw touching the table. The animal has then been moved slowly backward or forward (5 sec for 0.9 m) by the experimenter. The number of adjusting steps was counted for the right paw (33).

### Results

*In vivo* assays were carried out with drug combinations of the invention. Tested drug combinations induced a significant improvement either in the initiation time test or stepping test (table 5).

**Table 5**

| **Drug** Combination | **Protective effect against 6OHDA induced akinesia** |
|---|---|
| baclofen and torasemide | + |
| Baclofen - acamprosate - torasemide | + |
| mexiletine and cinacalcet | + |
| sulfisoxazole and torasemide | + |
| baclofen and acamprosate | + |
| acamprosate and cinacalcet | + |
| baclofen and cinacalcet | + |

As exemplified in figures 10 and 11, drug combinations of the invention strongly protect rats from 6OHDA stereotaxic lesions. Noteworthy, treatment with baclofen-acamprosate combination results in an almost full alleviation of akinesia in the stepping test, in a dose dependent manner.

### REFERENCES

1. de Lau, L.M. and M.M. Breteler, Epidemiology of Parkinson's disease. Lancet Neurol, 2006. 5(6): 525-35.
2. Samii, A., J.G. Nutt, and B.R. Ransom, Parkinson's disease. Lancet, 2004. 363(9423): p. 1783-93.
3. Savitt, J.M., V.L. Dawson, and T.M. Dawson, Diagnosis and treatment of Parkinson disease: molecules to medicine. J Clin Invest, 2006. 116(7): 1744-54.
4. Schapira, A.H. and P. Jenner, Etiology and pathogenesis of Parkinson's disease. Mov Disord, 2011. 26(6): 1049-55.
5. Gao, H.M. and J.S. Hong, Gene-environment interactions: key to unraveling the mystery of Parkinson's disease. Prog Neurobiol, 2011. 94(1): 1-19.
6. Abbott, A., levodopa: the story so far. Nature, 2010. 466(7310): S6-7.
7. Rascol O., Lozano A., Stern M., Poewe W., Milestones in Parkinson's disease therapeutics. Mov Disord, 2011. 26(6): 1072-82.
8. Obeso J.A., Rodriguez-Oroz M.C., Goetz C.G., Marin C., Kordower J.H., Rodriguez M., Hirsch E.C., Farrer M., Schapira A.H., Halliday G., Missing pieces in the Parkinson's disease puzzle. Nat Med, 2010. 16(6): 653-61.
9. Ettmayer, P., Amidon, G. L., Clement, B. & Testa, B. Lessons learned from marketed and investigational prodrugs. J. Med. Chem. 47, 2393-2404 (2004).
10. Beaumont, K., Webster, R., Gardner, I. & Dack, K. Design of ester prodrugs to enhance oral absorption of poorly permeable compounds: challenges to the discovery scientist. Curr. Drug Metab. 4, 461-485 (2003).
11. Heimbach T, Oh DM, Li LY, Rodríguez-Hornedo N, Garcia G, Fleisher D. Enzyme-mediated precipitation of parent drugs from their phosphate prodrugs. Int. J. Pharm. 261, 81-92 (2003).
12. Yang, C. Y., Dantzig, A. H. & Pidgeon, C. Intestinal peptide transport systems and oral drug availability. Pharm. Res. 16, 1331-1343 (1999).
13. Steffansen B., Nielsen C.U., Brodin B., Eriksson A.H., Andersen R., Frokjaer S., Intestinal solute carriers: an overview of trends and strategies for improving oral drug absorption. Eur. J. Pharm. Sci. 21, 3-16 (2004).
14. Stella , Borchardt R, Hageman M, Oliyai R, Maag H, Tilley J, Prodrugs: Challenges and Rewards. Vol. Vol. 1-2. 2007, New York: AAPS Press and Springer.
15. Wermuth, CG. The Practice of Medicinal Chemistry. (Hardbound, 2003). Part VI, Chap 33: Designing prodrugs and bioprecursors.
16. Pezron, I., Mitra A.K., Duvvuri S., Tirucherai G.S., Prodrug strategies in nasal drug delivery. Expert Opin. Ther. Pat., Vol. 12, No. 3, 331-340 (2002).
17. Stella, V. J. Prodrugs as therapeutics. Expert Opin. Ther. Pat. 14, 277-280 (2004).
18. Stella, V. J. & Nti-Addae, K. W. Prodrug strategies to overcome poor water solubility. Adv. Drug Deliv. Rev. 59, 677-694 (2007).
19. Higuchi, T.; Stella, V. eds. Prodrugs As Novel Drug Delivery Systems. ACS Symposium Series. American Chemical Society: Washington, DC (1975). 31.
20. Roche, E. B. Design of Biopharmaceutical Properties through Prodrugs and Analogs. American Pharmaceutical Association: Washington, DC (1977).
21. Lal R., Sukbuntherng J., Tai E. H., Upadhyay S., Yao F., Warren M. S., Luo W., Bu L., Nguyen S., Zamora J., Peng G., Dias T., Bao Y., Ludwikow M., Phan T., Scheuerman R. A., Yan H., Gao M., Wu Q. Q., Annamalai T., Raillard S. P., Koller K., Gallop M. A., Cundy K. C., Arbaclofen placarbil, a novel R-baclofen prodrug: improved absorption, distribution, metabolism, and elimination properties compared with R-baclofen. J Pharmacol Exp Ther, 2009. 330(3): 911-21.
22. Feng Xu, Ge Peng, Thu Phan, Usha Dilip, Jian Lu Chen, Tania Chernov-Rogan, Xuexiang Zhang, Kent Grindstaff, Thamil Annamalai, Kerry Koller, Mark A. Gallop, David J. Wustrow, Discovery of a novel potent GABAB receptor agonist; Bioorg Med Chem Lett. 2011 Nov 1; 21(21): 6582-5.
23. Andrew R. Leach,Valerie J. Gillet. An Introduction to Chemoinformatics. Springer 2007.
24. S. Asad Rahman, M. Bashton, G. L. Holliday, R. Schrader and J. M. Thornton: Small Molecule Subgraph Detector (SMSD) Toolkit, Journal of Cheminformatics 2009, 1:12.
25. Stahl H., Wermuth C. G. (Eds.) Handbook of Pharmaceutical Salts: Properties, Selection, and Use. Wiley-VCH; 2 edition (March 29, 2011).
26. Hanafi R, Mosad S, Abouzid K, Niess R, Spahn-Langguth H Baclofen ester and carbamate prodrug candidates: a simultaneous chromatographic assay, resolution optimized with DryLab.. J Pharm Biomed Anal. 2011 Nov 1; 56(3): 569-76.
27. Neugebauer G, Besenfelder E, von Möllendorff E. Pharmacokinetics and metabolism of torasemide in man. Arzneimittelforschung. 1988 Jan; 38(1A): 164-6.
28. Kahle PJ, Waak J, Gasser T. DJ-1 and prevention of oxidative stress in Parkinson's disease and other age-related disorders. Free Radic Biol Med. 2009 Nov 15; 47(10): 1354-61.
29. Lee DW, Rajagopalan S, Siddiq A, Gwiazda R, Yang L, Beal MF, Ratan RR, Andersen JK. Inhibition of prolyl hydroxylase protects against 1-methyl-4-phenyl-1,2,3,6-tetrahydropyridine-induced neurotoxicity: model for the potential involvement of the hypoxia-inducible factor pathway in Parkinson disease. J Biol Chem. 2009 Oct 16; 284(42): 29065-76.
30. Singer CA, Figueroa-Masot XA, Batchelor RH, and Dorsa DMMitogen-activated protein kinase pathway mediates estrogen neuroprotection after glutamate toxicity in primary cortical neurons. J. Neuroscience, 1999. 19(7):2455-2463.
31. Schinelli S, Zuddas A, Kopin IJ, Barker JL, di Porzio U. 1-Methyl-4-phenyl-1,2,3,6-tetrahydropyridine metabolism and 1-methyl-4-phenylpyridinium uptake in dissociated cell cultures from the embryonic mesencephalon. J Neurochem. 1988 Jun; 50(6): 1900-7.
32. Jouve L, Salin P, Melon C, Kerkerian-Le Goff L. Deep brain stimulation of the center median-parafascicular complex of the thalamus has efficient anti-parkinsonian action associated with widespread cellular responses in the basal ganglia network in a rat model of Parkinson's disease. J Neurosci. 2010 Jul 21; 30(29): 9919-28.
33. Olsson M, Nikkhah G, Bentlage C, Björklund A. Forelimb akinesia in the rat Parkinson model: differential effects of dopamine agonists and nigral transplants as assessed by a new stepping test. J Neurosci. 1995 May; 15(5 Pt 2): 3863-75.
34. Linazasoro G, Van Blercom N, Ugedo L & Ruiz Ortega JA. Pharmacological treatment of Parkinson's disease: life beyond dopamine D2/D3 receptors? J. Neural Transm. 2008 115, 431-41.
35. Jimenez-Shahed J., Hunter C.. Jankovic J. Acamprosate improves impulse control disorders in Parkinson's disease. Neurology 2008 March; 70 (11 Suppl.1): A288.
36. Fox, S. H., Brotchie, J. M., & Lang, A. E.. Non-dopaminergic treatments in development for Parkinson's disease. The Lancet. Neurology, 2008 October; 7(10), 927-38.
37. Madan A & Schiess MC. Intrathecal baclofen therapy slows progressive disability in multiple system atrophy. Neuromodulation 14, 176-8.

## Claims

1. A composition for use in the treatment of Parkinsonism, said composition comprising acamprosate and baclofen, or salts thereof, or a sustained release formulation thereof.

2. A composition for use of claim 1, wherein parkinsonism is a condition selected from Parkinson's disease, progressive supranuclear palsy, multiple system atrophy, cortical-basal ganglionic degeneration, diffuse Lewy body disease, Parkinson's dementia, X-linked dystonia-parkinsonism, and secondary parkinsonism.

3. The composition for use of claim 1, wherein the Parkinsonism is Parkinson's disease.

4. The composition for use of claim 1 or 3, comprising at least one of the following drug combinations:
- baclofen and acamprosate and cinacalcet,
- baclofen and acamprosate and torasemide, or
- baclofen and acamprosate and mexiletine,
or salts thereof, or a sustained release formulation thereof.

5. The composition for use of any one of claims 1-4, further comprising at least one dopamine precursor, at least one dopamine receptor agonist, and/or at least one inhibitor of dopamine metabolizing enzymes.

6. The composition for use of claim 5, wherein the at least one dopamine precursor is selected from the group consisting of levodopa and melevodopa, or salts or sustained release formulations thereof, the at least one dopamine receptor agonist is selected from the group consisting of talipexole, piribedil, rotigotine, bromocriptine, pergolide, cabergoline, lisuride, pramipexole, ropinirole or apomorphine, or salts or sustained release formulations thereof, and the at least one inhibitor of dopamine metabolizing enzymes is selected from the group consisting of selegiline and rasagiline, or salts or sustained release formulations thereof.

7. The composition for use of claim 5 or 6 comprising at least one of the following drug combinations, or salts or sustained release formulations thereof:
- baclofen and acamprosate and levodopa,
- baclofen and acamprosate and cinacalcet and levodopa,
- baclofen and acamprosate and torasemide and levodopa, or
- baclofen and acamprosate and mexiletine and levodopa.

8. The composition for use of claim 7, further comprising carbidopa or a salt thereof or a sustained release formulation thereof.

9. The composition for use of any one of the preceding claims, which further comprises a pharmaceutically acceptable carrier or excipient.

10. The composition for use of any one of the preceding claims, wherein the compounds are formulated or administered together, separately or sequentially.

11. The composition for use of any one of the preceding claims, wherein said composition is administered repeatedly to the subject.

12. The composition for use of any one of the preceding claims, wherein said composition is administered orally.

13. The composition for use of any one of claims 1 to 12, wherein acamprosate, is administered in a dosage of less than 50 mg per day, more preferably less than 10 mg per day.

14. The composition for use of any one of claims 1 to 13, wherein baclofen is administered in a dosage of less than 30 mg per day.

15. The composition for use of any one of claims 1 to 14, for use in protecting dopaminergic neurons of the nigrostriatal system from degeneration.

16. The composition for use of any one of claims 1 to 14, for use in the treatment of bradykinesia or akinesia.

17. The composition for use of any one of the preceding claims, for use in a preventive treatment in a subject in need thereof and at risk of Parkinsonism.

18. A composition comprising baclofen, acamprosate and levodopa, or any pharmaceutically acceptable salts thereof.

19. The composition of claim 18, further comprising carbidopa or a pharmaceutically acceptable salt or sustained release formulation thereof.

## Patentansprüche

1. Eine Zusammensetzung zur Verwendung in der Behandlung von Parkinsonismus, die Zusammensetzung umfassend Acamprosat und Baclofen oder Salze davon oder eine Retardformulierung davon.

2. Eine Zusammensetzung zur Verwendung nach Anspruch 1, wobei Parkinsonismus ein Zustand ist ausgewählt aus Parkinson-Krankheit, Progressive Supranukleäre Blickparese, Multisystematrophie, Corticobasalganglionäre Degeneration, diffuse Lewy-Körperchen-Erkrankung, Parkinson-Demenz, X-gekoppelter Dystonie-Parkinsonismus und sekundärer Parkinsonismus.

3. Die Zusammensetzung zur Verwendung nach Anspruch 1, wobei der Parkinsonismus Parkinson-Krankheit ist.

4. Die Zusammensetzung zur Verwendung nach Anspruch 1 oder 3, umfassend mindestens eine der folgenden Arzneimittelkombinationen:
- Baclofen und Acamprosat und Cinacalcet,
- Baclofen und Acamprosat und Torasemid oder
- Baclofen und Acamprosat und Mexiletin oder Salze davon oder eine Retardformulierung davon.

5. Die Zusammensetzung zur Verwendung nach einem der Ansprüche 1 bis 4, ferner umfassend mindestens ein Dopaminvorläufer, mindestens ein Dopaminrezeptoragonist und/oder mindestens ein Inhibitor der Dopamin-metabolisierenden Enzyme.

6. Die Zusammensetzung zur Verwendung nach Anspruch 5, wobei der mindestens eine Dopaminvorläufer ausgewählt ist aus der Gruppe bestehend aus Levodopa und Melevodopa oder Salze oder Retardformulierungen davon, der mindestens eine Dopaminrezeptoragonist ist ausgewählt aus der Gruppe bestehend aus Talipexol, Piribedil, Rotigotin, Bromocriptin, Pergolid, Cabergolin, Lisurid, Pramipexol, Ropinirol oder Apomorphin oder Salze oder Retardformulierungen davon, und der mindestens eine Inhibitor der Dopamin-metabolisierenden Enzyme ist ausgewählt aus der Gruppe bestehend aus Selegilin und Rasagilin oder Salze oder Retardformulierungen davon.

7. Die Zusammensetzung zur Verwendung nach Anspruch 5 oder 6 umfassend mindestens eine der folgenden Arzneimittelkombinationen oder Salze oder Retardformulierungen davon:
- Baclofen und Acamprosat und Levodopa,
- Baclofen und Acamprosat und Cinacalcet und Levodopa,
- Baclofen und Acamprosat und Torasemid und Levodopa oder
- Baclofen und Acamprosat und Mexiletin und Levodopa.

8. Die Zusammensetzung zur Verwendung nach Anspruch 7, ferner umfassend Carbidopa oder ein Salz davon oder eine Retardformulierung davon.

9. Die Zusammensetzung zur Verwendung nach einem der vorhergehenden Ansprüche, die ferner einen pharmazeutisch verträglichen Träger oder Exzipienten umfasst.

10. Die Zusammensetzung zur Verwendung nach einem der vorhergehenden Ansprüche, wobei die Verbindungen zusammen, getrennt oder nacheinander formuliert oder verabreicht werden.

11. Die Zusammensetzung zur Verwendung nach einem der vorhergehenden Ansprüche, wobei die Zusammensetzung wiederholt an das Subjekt verabreicht wird.

12. Die Zusammensetzung zur Verwendung nach einem der vorhergehenden Ansprüche, wobei die Zusammensetzung oral verabreicht wird.

13. Die Zusammensetzung zur Verwendung nach einem der Ansprüche 1 bis 12, wobei Acamprosat in einer Dosis von weniger als 50 mg pro Tag, mehr bevorzugt weniger als 10 mg pro Tag verabreicht wird.

14. Die Zusammensetzung zur Verwendung nach einem der Ansprüche 1 bis 13, wobei Baclofen in einer Dosis von weniger als 30 mg pro Tag verabreicht wird.

15. Die Zusammensetzung zur Verwendung nach einem der Ansprüche 1 bis 14 zur Verwendung zum Schutz dopaminerger Neuronen des nigrostriatalen Sytems vor Degeneration.

16. Die Zusammensetzung zur Verwendung nach einem der Ansprüche 1 bis 14, zur Verwendung in der Behandlung von Bradykinese oder Akinese.

17. Die Zusammensetzung zur Verwendung nach einem der vorhergehenden Ansprüche zur Verwendung in einer präventiven Behandlung in einem Subjekt, das sie benötigt und gefährdet für Parkinsonismus ist.

18. Eine Zusammensetzung umfassend Baclofen, Acamprosat und Levodopa oder jede pharmazeutisch verträglichen Salze davon.

19. Die Zusammensetzung nach Anspruch 18, ferner umfassend Carbidopa oder ein pharmazeutisch verträgliches Salz oder Retardformulierung davon.

## Revendications

1. Composition pour son utilisation dans le traitement du Parkinsonisme, ladite composition comprenant de l'acamprosate et du baclofène, ou des sels de ceux-ci, ou des formulations à libération prolongée de ceux-ci.

2. Composition pour son utilisation selon la revendication 1, ledit Parkinsonisme étant une maladie choisie parmi la maladie de Parkinson, la paralysie supranucléaire progressive, l'atrophie multisystématisée, la dégénérescence ganglionnaire corticale-basale, la maladie diffuse à corps de Lewy, la démence associée à la maladie de Parkinson, la dystonie avec parkinsonisme liée à l'X, et le parkinsonisme secondaire.

3. Composition pour son utilisation selon la revendication 1, ledit Parkinsonisme étant la maladie de Parkinson.

4. Composition pour son utilisation selon la revendication 1 ou 3, comprenant au moins l'une des combinaisons suivantes de médicaments:
- baclofène et acamprosate et cinacalcet,
- baclofène et acamprosate et torasemide, ou
- baclofène et acamprosate et mexiletine,
ou des sels de ceux-ci, ou des formulations à libération prolongée de ceux-ci.

5. Composition pour son utilisation selon l'une quelconque des revendications 1 à 4, comprenant en outre au moins un précurseur de la dopamine, au moins un agoniste des récepteurs de la dopamine, et/ou au moins un inhibiteur d'enzymes métabolisant la dopamine.

6. Composition pour son utilisation selon la revendication 5, dans laquelle le au moins un précurseur de la dopamine est choisi parmi le groupe constitué de lévodopa et melevodopa, ou des sels ou des formulations à libération prolongée de celles-ci, le au moins un agoniste des récepteurs de la dopamine est choisi dans le groupe constitué de talipexole, piribédil, rotigotine, bromocriptine, pergolide, cabergoline, lisuride, pramipexole, ropinirole ou apomorphine, ou des sels ou des formulations à libération prolongée de ceux-ci, et le au moins un inhibiteur d'enzymes métabolisant la dopamine est choisi dans le groupe constitué de selegiline et rasagiline, ou des sels ou des formulations à libération prolongée de celles-ci.

7. Composition pour son utilisation selon la revendication 5 ou 6, comprenant au moins l'une des combinaisons suivantes de médicaments, ou des sels ou des formulations à libération prolongée de ceux-ci:
- baclofène et acamprosate et lévodopa,
- baclofène et acamprosate et cinacalcet et lévodopa,
- baclofène et acamprosate et torasemide et lévodopa, ou
- baclofène et acamprosate et mexiletine et lévodopa.

8. Composition pour son utilisation selon la revendication 7, comprenant en outre la carbidopa ou un sel de celle-ci ou une formulation à libération prolongée de celle-ci.

9. Composition pour son utilisation selon l'une quelconque des revendications précédentes, comprenant en outre un support ou excipient pharmaceutiquement acceptable.

10. Composition pour son utilisation selon l'une quelconque des revendications précédentes, dans laquelle les composés sont formulés ou administrés ensemble, séparément ou séquentiellement.

11. Composition pour son utilisation selon l'une quelconque des revendications précédentes, ladite composition étant administrée de façon répétée au sujet.

12. Composition pour son utilisation selon l'une quelconque des revendications précédentes, ladite composition étant administrée oralement.

13. Composition pour son utilisation selon l'une quelconque des revendications 1 à 12, dans laquelle l'acamprosate est administré dans un dosage de moins de 50 mg par jour, de préférence moins de 10 mg par jour.

14. Composition pour son utilisation selon l'une quelconque des revendications 1 à 13, dans laquelle le baclofène est administré dans un dosage de moins de 30 mg par jour.

15. Composition pour son utilisation selon l'une quelconque des revendications 1 à 14, pour une utilisation dans la protection des neurones dopaminergiques du système nigrostratial contre la dégénérescence.

16. Composition pour son utilisation selon l'une quelconque des revendications 1 à 14, pour une utilisation dans le traitement de la bradykinésie ou akinésie.

17. Composition pour son utilisation selon l'une quelconque des revendications précédentes, pour une utilisation dans le traitement préventif chez un sujet le nécessitant et présentant un risque du Parkinsonisme.

18. Composition comprenant du baclofène, de l'acamprosate et de la lévodopa, ou des sels pharmaceutiquement acceptables de ceux-ci.

19. Composition selon la revendication 18, comprenant en outre la carbidopa ou un sel pharmaceutiquement acceptable de celle-ci ou une formulation à libération prolongée de celle-ci.
